# EUROPEAN PATENT APPLICATION

(11) **EP 2 742 974 A1**
(43) Date of publication of application: **18.06.2014**
(21) Application number: 12197087.5
(22) Date of filing: 13.12.2012
(51) Int. Cl.: A61P 21/00, A61P 25/14, A61K 31/137, A61K 31/14, A61K 31/36, A61K 31/435, A61K 31/4355, C07D 221/18, C07C 217/60

(54) **Phenanthrene derivatives for use as medicaments**

(71) Applicant: Valentia Biopharma, 46980 Valencia (ES)
(72) Inventor: García Alcover, Irma, 46980 Valencia (ES); López Catel, Arturo, 46980 Valencia (ES); Álvarez Abril, María del Carmen, 46980 Valencia (ES); Tormo Beltrán, Jose Rubén, 28002 Madrid (ES); Pérez Alonso, Manuel, 46010 Valencia (ES); Artero Allepuz, Rubén, 46010 Valencia (ES); Cortés Martínez, Diego Miguel, 46980 Valencia (ES)
(74) Representative: Illescas, Manuel

(57) **Abstract**

Phenanthrene derivatives of formula I for use as medicaments. The present invention refers to phenanthrene derivatives for use as medicaments, mainly in the prevention and/or treatment of DM1, HDL2, SCA8, DM2, SCA3, FXTAS, FTD/ALS, and SCA31. In a preferred embodiment, phenanthrene derivatives of the invention are also used as antimyotonic agents.

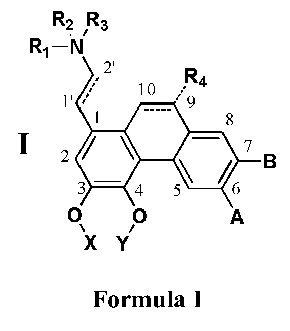

## Description

### FIELD OF THE INVENTION

The present invention refers to phenanthrene derivatives for use as medicaments, mainly in the prevention and/or treatment of human diseases involving presence of toxic RNAs comprising: Myotonic Dystrophy Type 1 (DMI), Huntington's Disease Like 2 (HDL2), Spinocerebellar Ataxia Type 8 (SCA8), Myotonic Dystrophy Type 2 (DM2), Spinocerebellar Ataxia Type 3 (SCA3), Fragile-X-Associated Tremor/Ataxia Syndrome (FXTAS), Frontotemporal Degeneration/Amyotrophic Lateral Sclerosis (FTD/ALS), and Spinocerebellar Ataxia Type 31 (SCA31). In a preferred embodiment, said phenanthrene derivatives are used as antimyotonic agents. Therefore, this invention may be included either in the whole pharmaceutical or medical field.

### STATE OF THE ART

DM1, also called *Dystrophia Myotonica* or Steinert's disease, was described more than 100 years ago. DM1 displays an autosomal dominant mode of inheritance and is now recognized as one of the most common forms of muscular dystrophy in adults with a prevalence of 1:8,000-20,000 individuals worldwide (Harper PS. Major Problems in Neurology: Myotonic Dystrophy. London, UK: WB Saunders (2001*)).* The name myotonic dystrophy refers to two main muscle symptoms identified in this disease: myotonia refers to a state of delay in muscle relaxation, while dystrophy indicates the progressive deterioration of muscle. In addition to muscular dystrophy and myotonia, DM1 includes a consistent group of apparently unrelated and rare clinical symptoms, like: cardiac conduction defects, insulin resistance, ocular cataracts, cognitive abnormalities (developmental delays and learning problems), daytime sleepiness and testicular atrophy. Due to said diversity in symptoms, this disease is perhaps best classified as a multisystemic neuromuscular disease. It is important to emphasize that not all the above cited problems occur in a single patient because DM1 is not only highly variable in the type of symptoms, but also severity and age of onset strongly differ.

In 1992, the genetic mutation underlying DM1 (OMIM #160900) was identified as an expanding (CTG)n repeat within the DM1 locus in the 3' untranslated region of the DMPK (Dystrophy Myotonic Protein Kinase) gene on chromosome 19q13.3 (Brook J. et al. Cell, 68:799-808 (1992*)).* Manifestation of disease generally correlates with (CTG)n repeat length, as determined in blood DNA. Number of repeats in the DMPK gene tends to increase from generation to generation, accounting for the genetic anticipation (i.e. the earlier onset of disease manifestation in successive generations) in DM1 families. Next to intergenerational CTG-triplet repeat instability, somatic instability in tissues is also observed during adulthood and ageing. Four categories of clinical manifestation of DM1 can be distinguished based on the number of CTG repeats: (i) normal or unaffected individuals carry a CTG repeat between 5 and 37 triplets; (ii) mild or late onset of disease is seen in patients with 50 to 150 triplets, most of these patients only have cataracts; (iii) classic disease manifestation is associated with repeat lengths of 100 to 1000 CTG triplets; and (iv) DM1 patients with congenital or juvenile onset disease carry several thousands of CTG triplets in their blood DNA. Molecular mechanism underlying DM1 pathogenesis implicates several cellular pathways and is not yet well established. However, the most widely accepted pathogenic pathway is a toxic RNA gain of function where repeat containing mRNAs form toxic dsRNA hairpins that are retained in nuclear foci. These hairpins sequester several nuclear factors, including the splicing factor Muscleblind-like 1 (MBNL1) (Jiang H et al. Hum. Mol. Genet. 13(24):3079-88 (2004*)).* Sequestration of MBNL1 has a key role in DM1 pathogenesis, since MBNL1 knockout (KO) mice showed much of the pathogenesis seen in CTG mouse models (Kanadia RN et al. Science, 302:1978-80 (2003*)),* and over-expression of MBNL1 in a DM1 mouse model is able to reduce disease symptoms (Kanadia RN et al. Proc. Natl. Acad. Sci. U S A, 103:11748-53 (2006*)).* Importantly, several mRNAs whose splicing is regulated by MBNL1 showed aberrant splicing in DM1 (Du H et al. Nat. Struct. Mol. Biol. 17(2):187-93 (2010*)).* Thus, DM1 has been defined as a spliceopathy, a disease mainly caused by splicing defects, and some of the most relevant symptoms in this disease have been related to splicing defects: aberrant exclusion of insulin receptor exon 11 originates a lower signaling receptor that probably causes insulin resistance; unusual inclusion of C1C-1 7a exon generates a STOP codon which triggers this mRNA to the Non Sense Mediated Decay pathway (Ranum LP and Cooper TA. Annu. Rev. Neurosci. 29:259-77(2006*));* and recently, muscle degeneration has been linked to aberrant splicing of bridging integrator-1 (BIN1) (Fugier C et al. Nat. Med. 17(6):720-5. (2011*)).*

Since the discovery that RNAs with CUG expansions may be toxic for cells, a whole series of scientific evidence supports the hypothesis that these expansions, or similar expansions, in other RNAs, may cause hereditary genetic pathologies similar to DM1. For example, in DM2, expansions of the CCUG tetranucleotide in the first intron of the RNAs transcribed from the CNBP (zinc-30 finger protein 9, Entrez #7555) gene trigger a pathology that is very similar to DM1 (OMIM #602668). In fact, in the state of the art, genes related to the nervous function present altered levels in both DM1 and DM2 patients, which supports the idea of a pathogenesis mechanism common to both diseases. This list is growing and includes diseases as some spinocerebellar ataxias (SCA8, OMIM #603680; SCA3 OMIM #109150, SCA10 OMIM #603516, SCA31 OMIM #117210, SCA36 OMIM #614153), Fragile X-associated tremor/ataxia syndrome (FXTAS, OMIM #300623), Huntington's disease-like 2 (HDL2, OMIM #606438) (reviewed in Dickson AM and Wilusz CJ. WIREs RNA 1:173-192 (2010*)),* and recently frontotemporal dementia and/or amyotrophic lateral sclerosis (FTD/ALS #105550) (DeJesus-Hernandez M et al. Neuron. 72(2):245-56 (2011*)).*

Although DM1 was first described in 1909, there is still not an effective therapy available. The mechanism of pathogenesis of the DM1 is nowadays reasonable well understood and several experimental therapies are being investigated in animal models of the disease. However, although all the treatments applied are palliative and contribute to mitigate the development of symptoms, in no case prevent the onset thereof or treat the disease in a definitive manner. For example, there is no compound capable of reverting lack of chloride conductance so as to reduce myotonia. Some compounds, such as mexiletine, quinidine, phenytoin, procainamide or carbamazepine, which inhibit the sodium entry necessary for the initiation and propagation of impulses, are administered to patients in order to treat this symptom. Occasionally, said molecules are used, in turn, as a treatment for cardiac arrhythmias; consequently, given the risk that they entail due to their effect on the cardiac function, it is preferable to avoid using them as anti-myotonic agents. Moreover, many of these treatments reduce muscular strength.

Consequently the present invention is focused on solving the above explained problems, providing chemical compounds or drugs acting as antimyotonic agents and also being effective in the treatment of human diseases involving the presence of toxic RNA comprising CTG (DM1, HDL2 and SCA8), CCTG (DM2), CAG (SCA3), CGG (FXTAS), GGGGCC (FTD/ALS), and TGGAA (SCA31) repeats.

### DESCRIPTION OF THE INVENTION

### Brief description of the invention

Such as it is explained above, the present invention refers to phenanthrene derivatives for use as medicaments, mainly in the prevention and/or treatment of human diseases with presence of pathologic toxic RNAs comprising DM1, HDL2, SCA8, DM2, SCA3, FXTAS, FTD/ALS and SCA31. In a preferred embodiment, phenanthrene derivatives of the invention are used as antimyotonic agents.

The compounds of the present invention are primarily based on the massive *in vivo* screening (*in vivo* High Throughput Screening (HTS)) of chemical libraries containing thousands of small molecules by using transgenic *Drosophila* models for DM1 disease as reading tools (Garcia-Lopez et al, 2008 and developed in house stocks). Results after primary screening of ∼15,000 compounds allowed describing several (more than 20) novel chemical entities (hits) with potential anti-DM1 candidates. The list of compounds of the present invention is built on further development of two specific hits (VLT002 and VLT015) identified with analogous chemical backbone. The present invention shows that these molecules display anti-DM1 activity after measurement on well-established DM1 phenotypes in different disease models (see figures and examples set below). At the same time, an interesting mechanism of action is suggested since some of the compounds have shown the ability to bind to the toxic double-stranded CUG-RNA hairpin and modify the structure preventing the aberrant binding of MBNL1 and potentially any other molecules whose binding might cause or intensify the pathological phenotype.

Thus, several compounds of Formula I (see below) has been identified in the present invention having therapeutic potential to prevent or treat DM1 which is caused by the presence of toxic RNA repeat transcripts that comprise a CUG motif. For this purpose, it was initially performed *in vivo* screening of chemical libraries in a *Drosophila* model of the disease. It was observed that the *Drosophila* model described in (Garcia-Lopez *et al.,* 2008), which expresses 480 CTG repeats (CTG(480)), displayed important DM1 disease phenotypes that respond to levels of Muscleblind and is susceptible to chemical modification, which makes this model suitable for the systematic search of compounds. Based on this, and developing novel in house *Drosophila* stocks, it was generated an automated minigene-based drug screening assay by measuring luciferase reporter in adult flies in order to accurately measure potential disease phenotype recovering after the treatment with the compounds of the invention. As explained above, the present invention begins with the screening of several chemical libraries (-15,000 small molecules), identification of more than 20 primary hits, and final selection and further development of two interesting ones (VLT002 and VLT015), which give rise to other interesting compounds supported throughout the present invention in terms of activity and mechanism of action. This approach ended with the recognition of a specific Markush formula (see Formula I below), and the assessment of the anti-DM1 activity of the compounds of the invention comprised in said Formula I and also of their capacity to improve or recover DM1 phenotypes characterized by disease hallmarks (precisely the aberrant aggregation of ribonuclear foci). The molecules comprised in the Formula I were further tested and validated in additional disease phenotypes in different models as *Drosophila,* human cells and mice. The mechanism of action suggested for the compounds of the present invention is linked to their ability to bind CUG repeat toxic structures and switching them to a non-toxic conformation, thereby preventing the aberrant binding or sequestration of MBNL and any other molecules whose binding to CUG hairpin loops could also cause or intensify the pathological phenotype.

On the other hand, the size of the molecules to be used in the present invention as therapeutic agents is an important issue to be considered, since a molecular weight that is too high limits the absorption of the compound inside the cell. The optimization of molecules to produce more active compounds is usually accompanied by an increase in the final size thereof. However, a molecular weight greater than 1,000 Daltons reduces the molecules' therapeutic potential, by reducing the bioavailability thereof, which makes it necessary to start from small compounds. Thus, 80% of the drugs currently commercialized have a molecular weight of less than 450 Daltons. All the specific molecules here identified have a molecular weight of 250 to 500 Daltons.

More precisely, the present invention refers to "phenanthrene-1-enthylamine" derivatives of Formula I 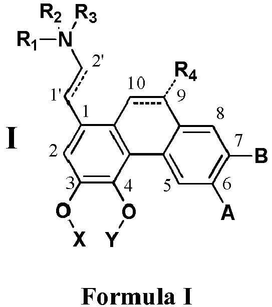 Wherein
**R₁, R₂** and **R₃** may be, independently of each compound: H, alkyl or benzyl radicals. In a preferred embodiment of the invention, **R₁**, **R₂** and **R₃** may be: H, CH₃, CH₂CH₃, CH₂CH₂CH₃, or Benzyl. **R₁**, **R₂** and **R₃** may be Carbon 10 (part of a cyclic ring with a possible subsequent lack of double bond C9-C10).
**R₄** may be, independently of each compound: H, a carbonyl or an alcohol. In a preferred embodiment of the invention, **R₄** may be: H, O or OH.
**X** and **Y** may be, independently of each compound: alkyl, aryl, benzyl, urethane or tiourethane radicals. In a preferred embodiment of the invention, **X** and **Y** may be: H, CH₂, CH₃, CH₂CH₃, Benzyl, C₂H₄Br, C₃H₆NO, C₇H₅NF or C₇H₃NSF₃. Or **X,Y** as part of a cyclic ring, including **X=Y** as a possibility.
**A** and **B** may be, independently of each compound: H, -OH, halides, alkyl-halides, -O-alkyl, -O-aryl, -O-benzyl, -O-urethane or -O-tiourethane radicals. In a preferred embodiment of the invention, **A** and **B** may be: H, OH, OCH₂O, Cl, OCH₃, OC₃H₇, OC₇H₇, OC₂H₄Br, OC₃H₆NO, OC₇H₅NF or OC₇H₃NSF₃. Or **A,B** as part of a cyclic ring, including **A=B** as a possibility.

The above cited possible **A, B**, **X** or **Y** moieties are represented as follows:

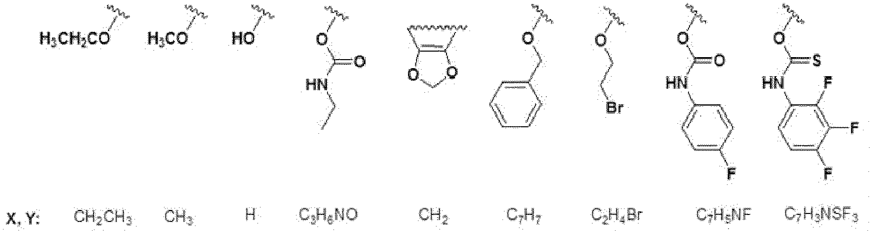

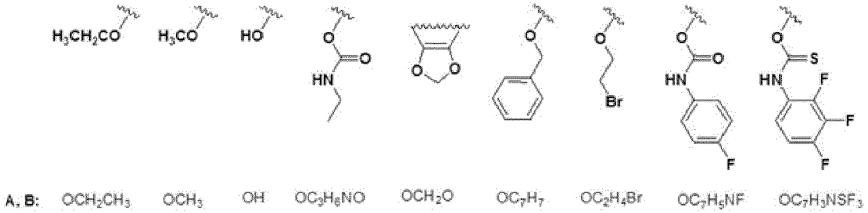

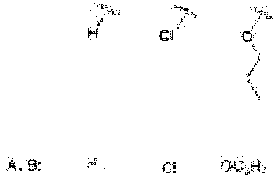

Intermittent/discontinuous bonds in Formula I mean that the bond may be present or not.

In a still preferred embodiment the present invention refers to the following compounds comprised in the above cited Formula I:
**VLT002:** R₁= R₂= CH₃; X,Y= CH₂; A= B= H 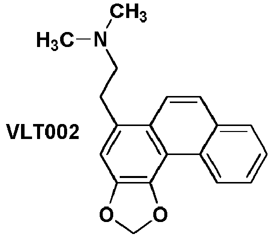
**VLT025:** R₁= R₂= R₃= CH₃; X,Y= CH₂; A= B= H 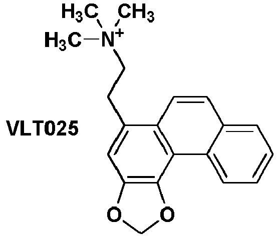
**VLT024:** R₁= R₂= R₃= CH₃; X= Y= CH₃; A= B= OCH₃ 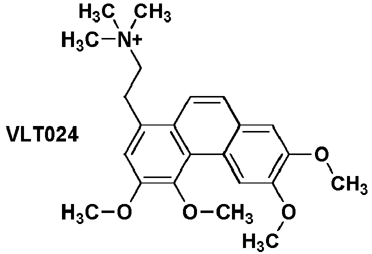
**VLT023:** R₁= R₂= CH₃; X= Y= CH₃; A= B= OCH₃ 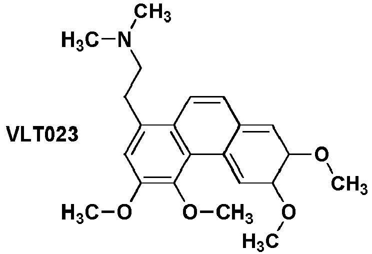
**VLT026:** R₁= CH₃; R₂= Bn; X= Bn; Y= CH₃; A= OCH₃; B= OBn 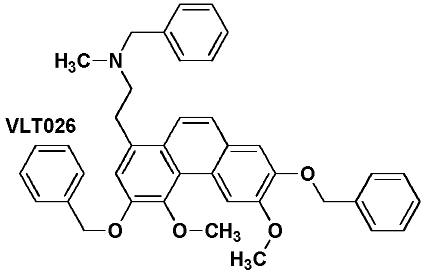
**VLT051:** R₁= R₂= R₃= CH₃; X= Y= CH₃; A= B= H 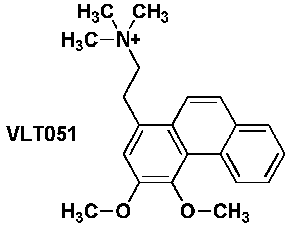
**VLT015:** R₁= CH₃; R₂= Carbon-10; X= H; Y= CH₃; A= OCH₃; B= OH 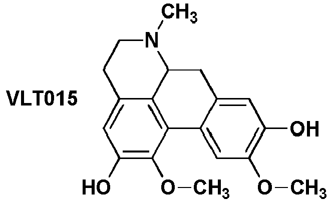
**VLT040:** R₁ = H; R₂= Carbon-10; X,Y= CH₂; A= B= H 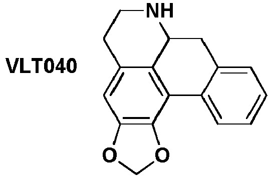
**VLT042:** R₁= R₂= CH₃; R₃= Carbon-10; X,Y= CH₂; A= B= H 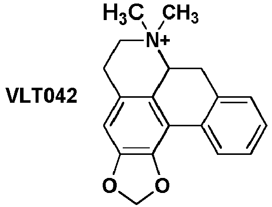
**VLT043:** R₁= CO-CH₃; R₂= Carbon-10; X,Y= CH₂; A= B= H 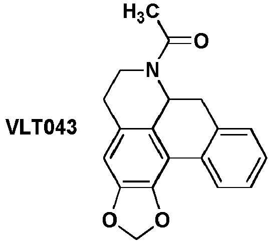
**VLT048:** R₁= CH₃; R₂= Carbon-10; X= CO-NH-CH₂-CH₃; Y= CH₃; A= OCH₃; B= OCONH-CH₂-CH₃ 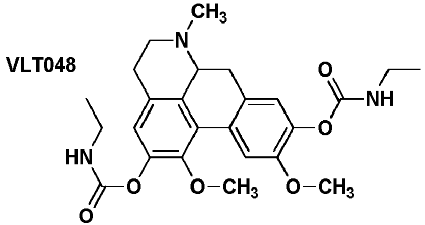
**VLT049:** R₁= CH₃; R₂= Carbon-10; X=Y= CH₃; A= B= H 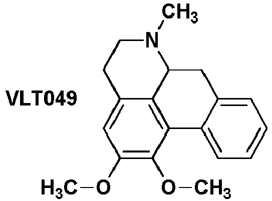
**VLT045:** R₁=R₂=Carbon-10; X,Y=CH₂; A=B=H; C₄=C₅; C₆₌C₆ₐ; C₇=O

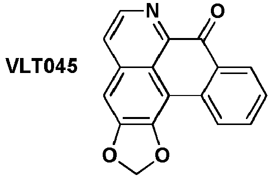

Consequently, the first embodiment of the present invention refers to compounds of Formula I, derivatives, or pharmaceutically acceptable salts thereof, for use as a medicament. In a preferred embodiment, the present invention refers to compounds of Formula I for use as an antimyotonic agent, preferably in the prevention and/or treatment of human diseases involving the presence of toxic RNA comprising: DM1, DM2, SCAs, FXTAS, HDL2 or FTD/ALS.

For the purpose of the present invention "antimyotonic agent" refers to any compound able to improve the slow relaxation of the muscles after voluntary contraction or electrical stimulation, a symptom of a small handful of certain neuromuscular disorders.

In a preferred embodiment, the compounds of Formula I for use as a medicament, or as an antimyotonic agent, preferably in the prevention and/or treatment of human diseases involving the presence of toxic RNA comprising: DM1, DM2, SCAs, FXTAS, HDL2 or FTD/ALS, are selected from: VLT002, VLT025, VLT024, VLT023, VLT026, VLT051, VLT015, VLT040, VLT042, VLT043, VLT048, VLT049 or VLT045. In a preferred embodiment, the compounds of Formula I for use as a medicament, or as an antimyotonic agent, preferably in the prevention and/or treatment of DM1, DM2, SCAs, FXTAS, HDL2 or FTD/ALS, are precisely selected from: VLT002, VLT025 or VLT015. In a still preferred embodiment the present invention refers to compounds of Formula I for use in the treatment of DM1, precisely selected from: VLT002, VLT025 or VLT015.

The second embodiment of the present invention refers to a pharmaceutical composition comprising a compound of Formula I as defined above and pharmaceutically acceptable carriers, for use as a medicament. In a preferred embodiment the present invention refers to a pharmaceutical composition comprising a compound of Formula I as defined above and pharmaceutically acceptable carriers for use as an antimyotonic agent, preferably for use in the treatment of DM1, DM2, SCAs, FXTAS, HDL2 or FTD/ALS.

The last embodiment of the present invention refers to the compounds of Formula I selected from: VLT026 or VLT048.

As cited above, compounds of Formula I can be administered either alone or formulated in pharmaceutical compositions which combine at least two compounds of Formula I. Alternatively, said composition may comprise at least one compound of Formula I and pharmaceutically acceptable carriers or excipients such as: binders, fillers, disintegrators, lubricants, coaters, sweeteners, flavouring excipients, colouring excipients, transporters, etc. or combinations thereof. Also, the compounds of Formula I can be part of pharmaceutical compositions in combination with other active ingredients (the latter not comprised in the Formula I).

On the other hand, the administration of the compounds of Formula I can be carried out by any means, for example enterally (IP), orally, rectally, topically, by inhalation or by intravenous, intramuscular or subcutaneous injection.

In addition, the administration may be either according to the Formula I above described or in any pharmaceutically acceptable salt, solvates or derivative thereof, such as: esters, ethers, alkyl, acyl, phosphate, sulfate, ethyl, methyl, propyl, salts, complexes, etc.

In a preferred embodiment, the compounds of the invention may be administered in the form of prodrugs as a pharmacological substance that is administered in an inactive (or less than fully active) form, and is subsequently converted to an active pharmacological agent (drug) through normal metabolic processes (bioactivation). These prodrugs are used to improve the absorption, distribution, metabolism and excretion of the active principle. Thus, said prodrugs are often designed to improve bioavailability or their selectively to interacts with the intended target. Particularly, in the present invention, the inclusion of carbamates or urethanes gives rise to prodrugs which, as explained above, improve the bioavailability of the compounds of Formula I.

Another embodiment of the present invention refers to a method for the prevention and/or treatment of human diseases involving presence of toxic RNAs comprising: DM1, HDL2, SCA8, DM2, CAG SCA3, FXTAS, FTD/ALS, and SCA31, which comprises the administration to the patients at least a compound of Formula I or a composition comprising thereof.

### Brief description of the figures

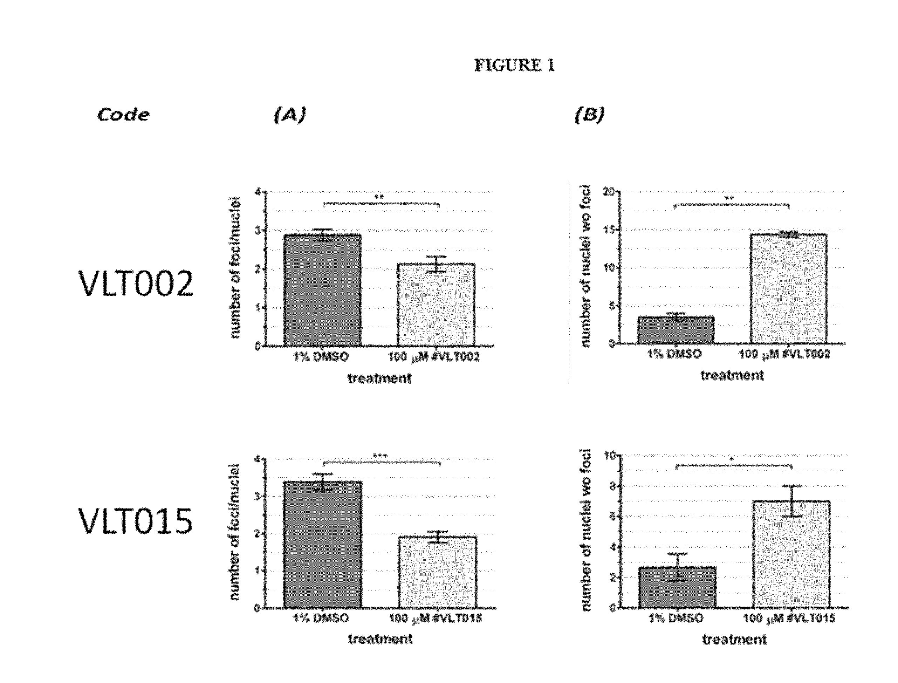

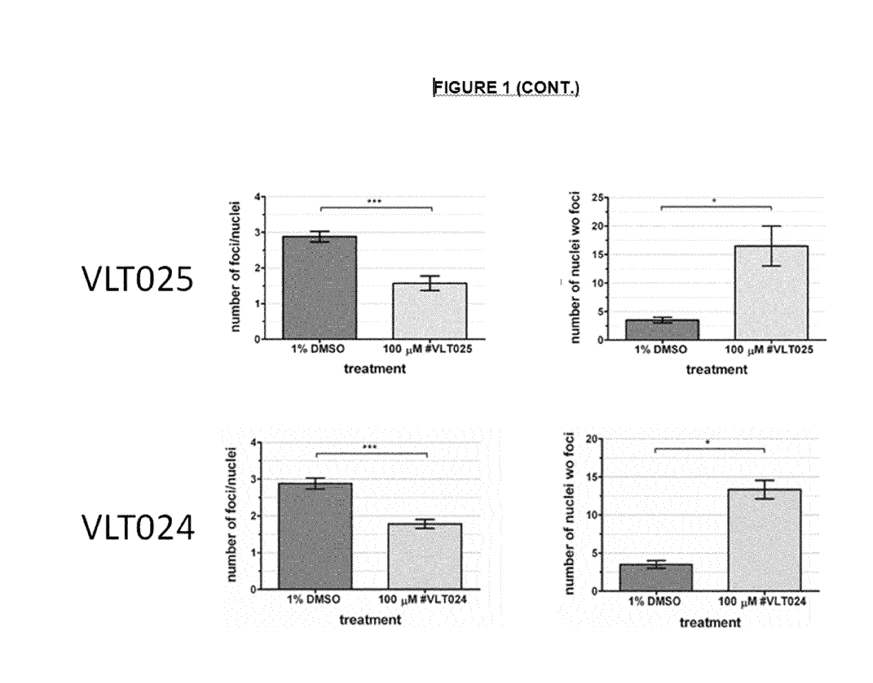

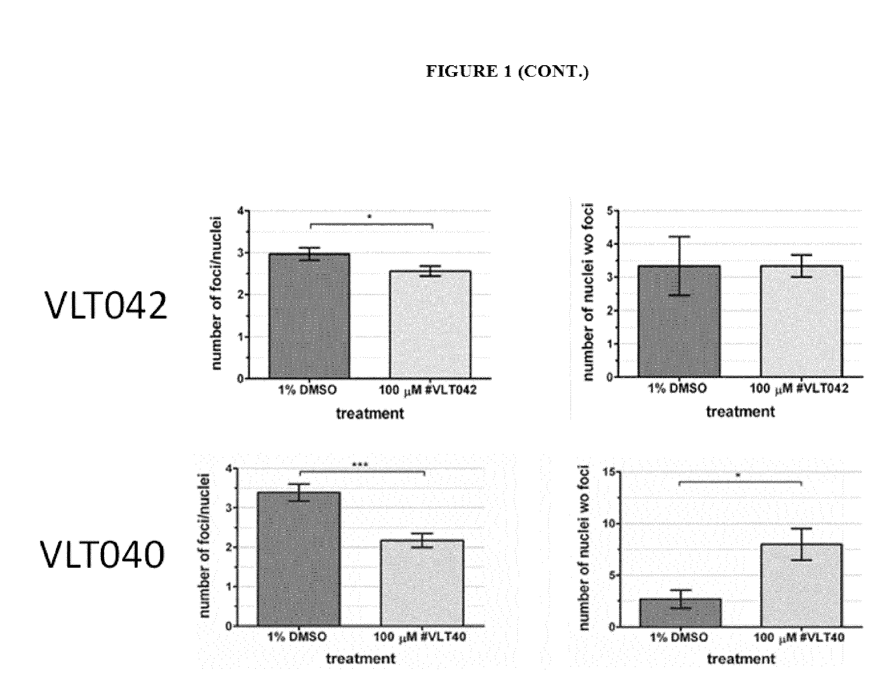

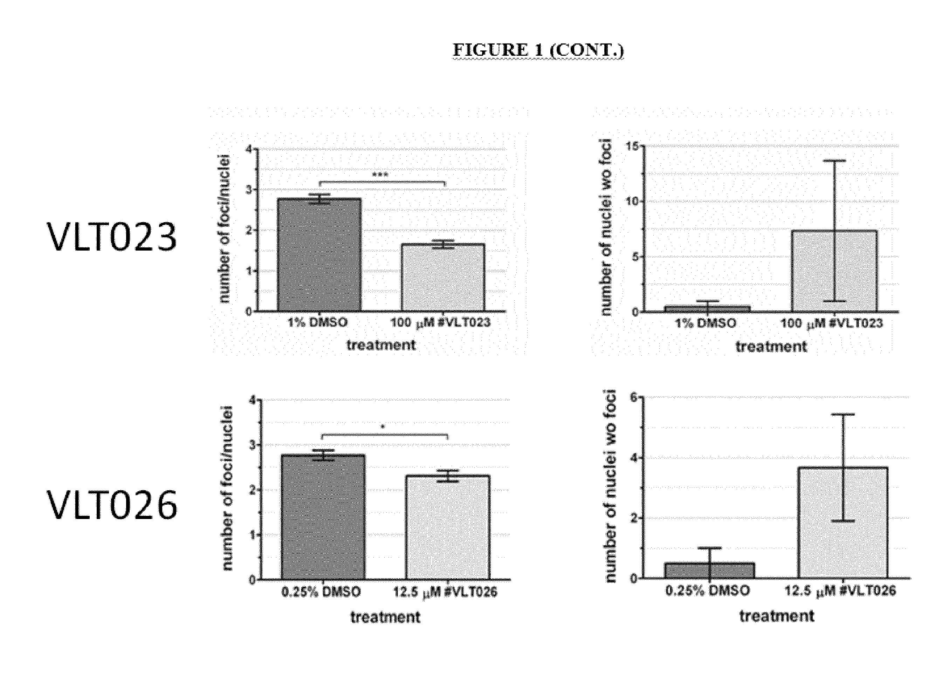

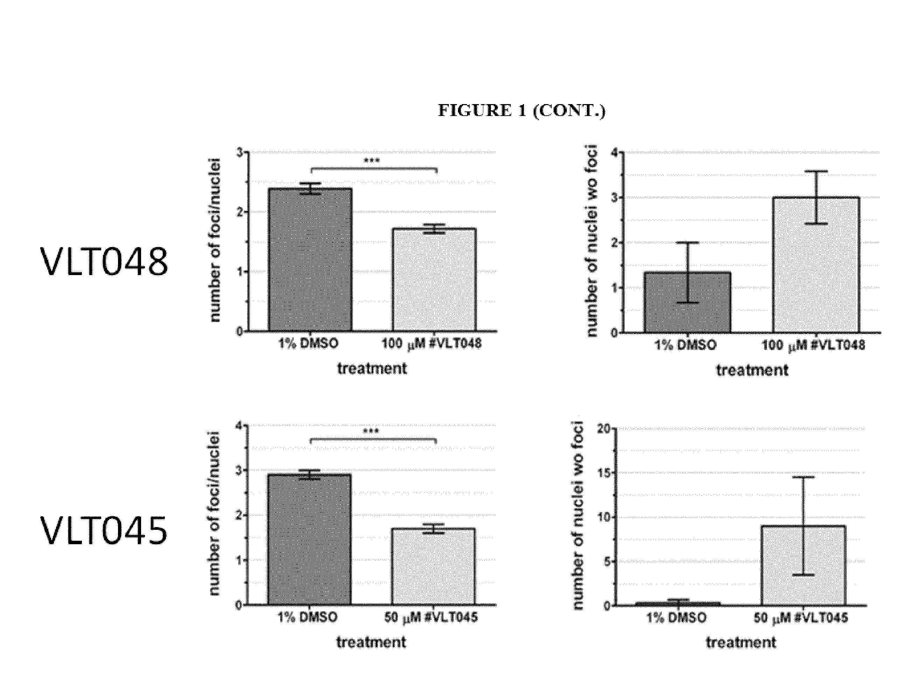

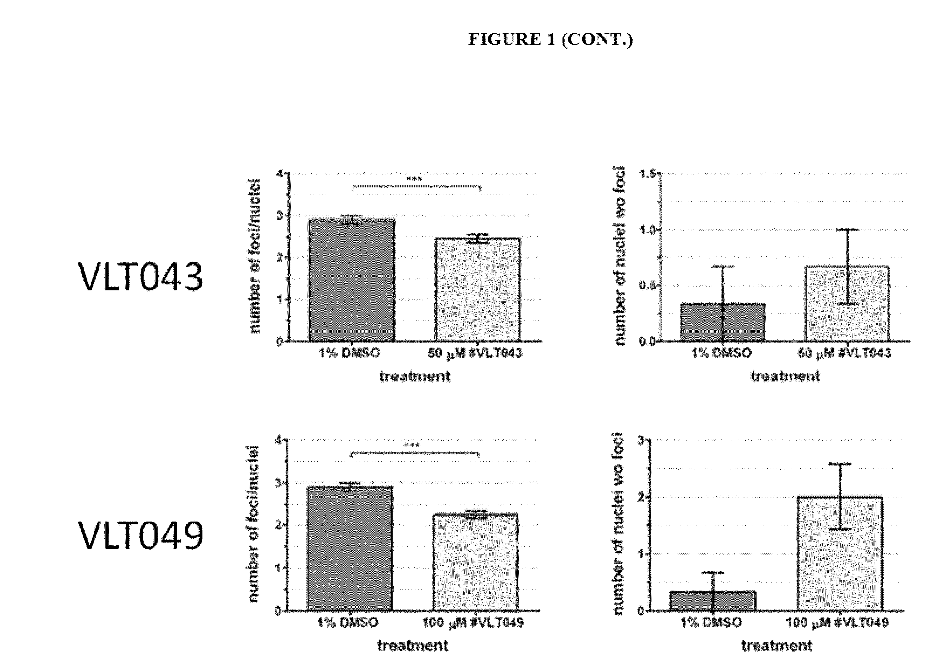

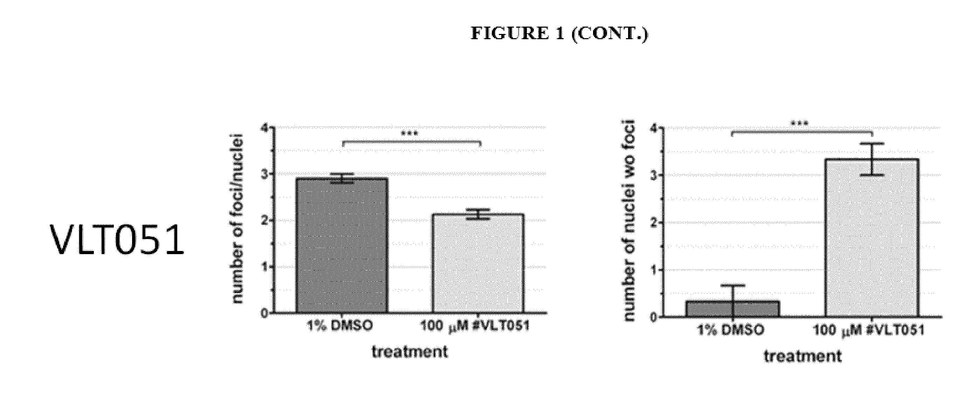

**Figure 1****.** Quantification of aberrant ribonuclear foci (DM1 disease hallmark) in human DM1 fibroblasts. Treatments with the compounds of Formula I were performed (VLT002, VLT015, VLT025, VLT024, VLT042, VLT040, VLT023, VLT026, VLT048, VLT045, VLT043, VLT049 and VLT051). **(A)** Number (medium) of foci/nuclei (No treatment vs. compound treatment). **(B)** Number of cells without foci (No treatment vs. compound treatment). n=100 minimum number of cells counted. T-Student test: * indicates p-value <0.05 ** p-value p<0.005 *** p-value p<0.0005. A significant reduction of this hallmark of DM1 disease was observed in at least one of the readings (A) or (B) performed for each compound tested.
   Such as it can be seen in this **Figure 1****,** all the assayed compounds of Formula I give rise to a statistically significant reduction in the number of foci/nuclei **(A),** and/or also to a statistically significant increase in the number of cells without foci **(B).**

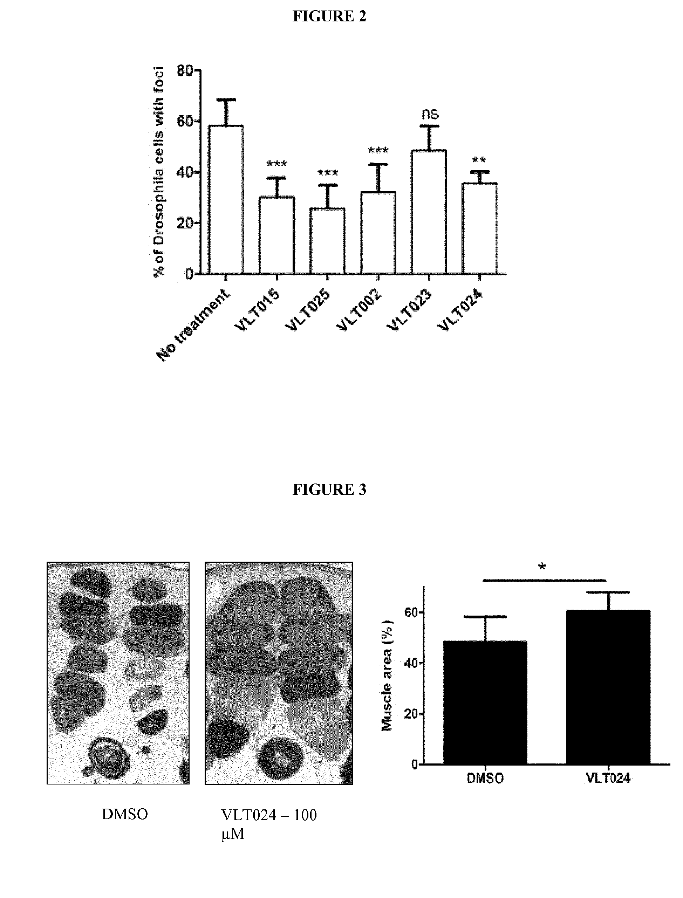

**Figure 2****.** Quantification of aberrant ribonuclear foci in a DM1 *Drosophila* model (MHC-Gal4/+;UAS-i480CTGs/+ flies; Garcia-Lopez A et al. PLoS One. 3(2):e1595 (2008*)).* Treatments with the compounds of Formula I (VLT015, VLT025, VLT002, VLT023, VLT024) comprised in the Formula I structure were performed. n=200 minimum number of cells counted. T-Student test: * indicates p-value <0.05 ** p-value <0.005 *** p-value p<0.0005.
   A strong reduction of the aberrant phenotype is observed even from those compounds without reaching a statistically significant value.
**Figure 3****.** Representative bright field microscopy images of transversal sections of resin-embedded adult IFM (indirect flight muscles) of MHC-Gal4/+;UAS-i480CTGs/+ flies that had been treated with DMSO (left) or with VLT024 (100 µM) (right).
   As can be seen, flies treated with VLT024 displayed a significant increase of the muscle area relative to DMSO-treated flies (quantified in graphic) * indicates p-value < 0.05. A minimum of 8 flies were analyzed in each case.

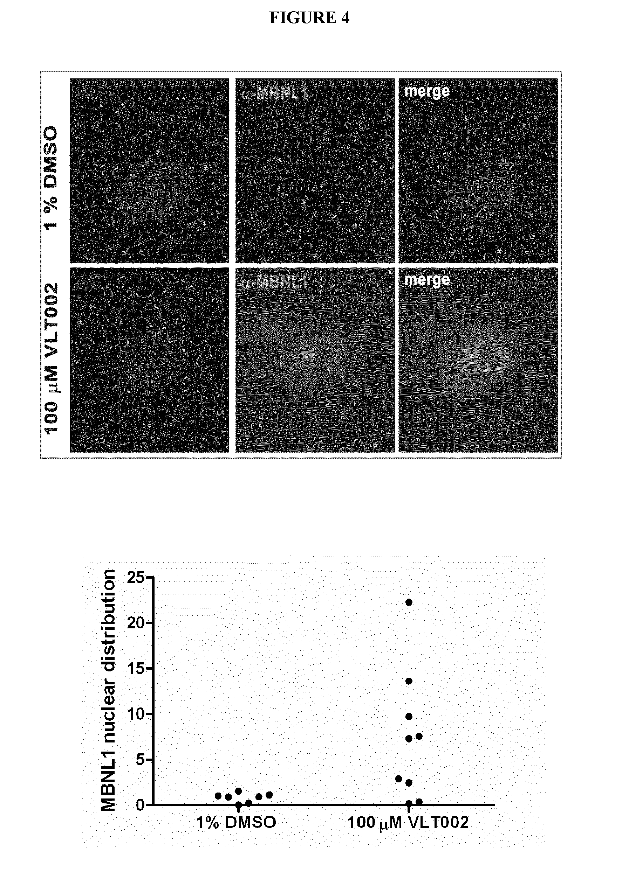

**Figure 4****.** VLT002 relieves MBNL1 sequestration. Upper row: Untreated DM1 fibroblasts with MBNL1 localizing as ribonuclear foci (see dots in middle and right pictures) to the nucleus (DAPI, detected as gray spherical shadow in left and right pictures). Bottom row: In same DM1 fibroblasts, VLT002 (100µM) reduced formation of the foci (no dots in middle and right pictures), MBNL1 sequestration is relieved (more intense gray in middle and right pictures). Bottom graphic: Quantification of the area (into the nucleus), where MBNL1 present (Photoshop software), reflects the MBNL1 release after VLT002 treatment.
   Consequently, VLT002 is able to effectively reduce the formation of foci and also to relieve MBNL1 sequestration.

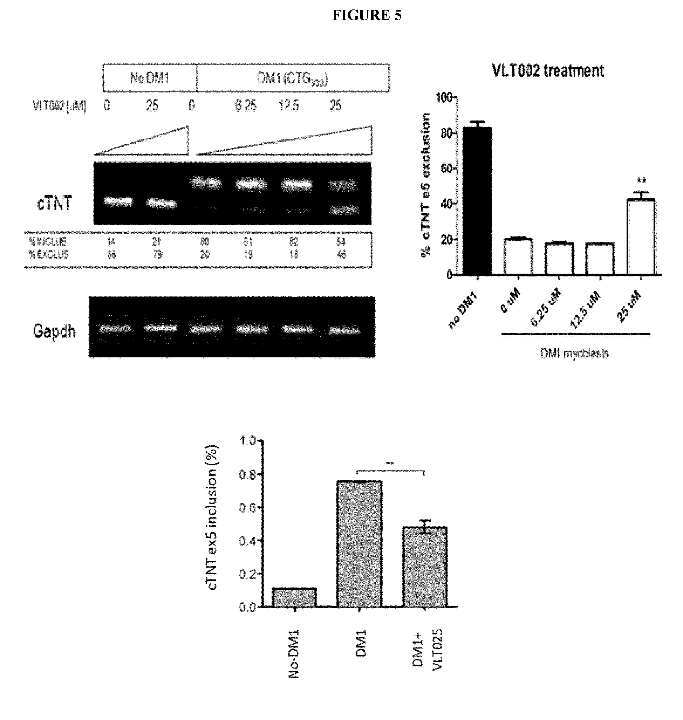

**Figure 5****.** Recovering of DM1 aberrant cTNT splicing event. Upper section, left: Dose-response treatments with VLT002 in human myoblasts displayed no changes on the high levels of exon 5 exclusion (normal) displayed by no DM1 cells. However, DM1 myoblasts display high levels of exon 5 inclusion (aberrant) and treatment with VLT002 showed partial recovering of normal cTNT splicing (ex5 exclusion) after 25 µM VLT002 treatment. No expression changes were observed after VLT002 treatment in a housekeeping gene (GAPDH). Upper section, right: Recovering was significant at 25 µM. Down section: Similar results were observed (in this case represented as ex5 inclusion) after VLT025 treatment at 25 µM. ** indicates p-value p<0.005.

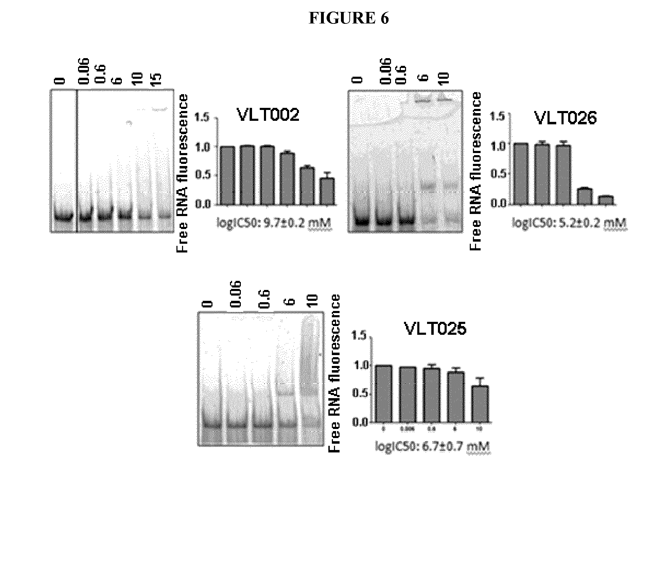

**Figure 6****.** Study of the binding of some compounds of Formula I (VLT002, VLT025 and VLT026) to FAM-(CUG)10 (repeat tract present in DM1, HDL2, SCA8 diseases) by means of gel retardation assays. VLT002, VLT025 and VLT026 bind to FAM-CUG10, causing retention of the complex in the well and displaying 50% binding (logIC50) at concentrations ranged between 5 and 10 (or 15 in VLT002) mM.

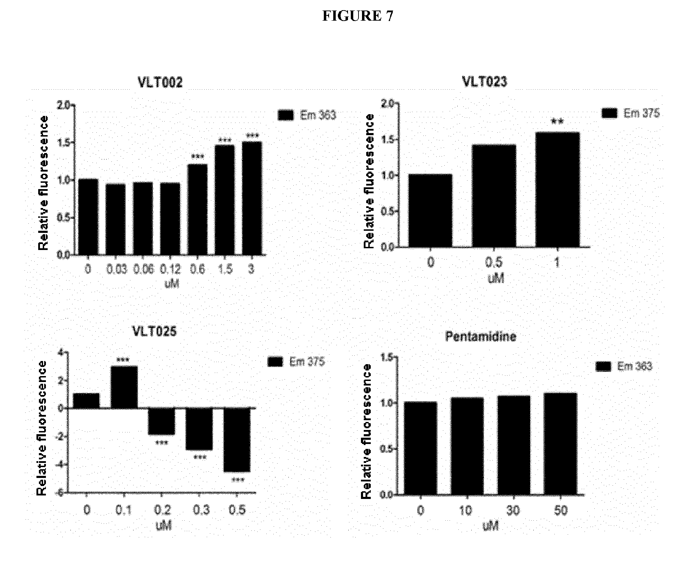

**Figure 7****.** VLT002, VLT023 and VLT025 modify the hairpin loops formed by the CUG repeats. Measurements of the fluorescence emitted (Y-axis), measured as relative fluorescence units (RFU), by 2-aminopurine in a (CUG)23 RNA (1 µM) in the presence of different compounds (dose-range approach), relative to the fluorescence emitted by the free RNA. All four compounds caused a >1.5 increase in the fluorescence of 2-AP at some point, which indicates a change towards single-stranded. Interestingly, VLT022 and VLT025 also showed noteworthy decreases in the fluorescence of 2-AP suggesting a change to a more structured and inflexible double-stranded. These effects were not observed when incubating the RNA with DMSO (not shown), or with the small molecule pentamidine, published as a compound able to disrupt MBNL1 binding to CUG hairpins (Warf MB et al. Proc. Natl. Acad. Sci. USA 106:18551-56 (2009*)).* * indicates p-value <0.05 ** p-value <0.005 *** p-value p<0.0005.

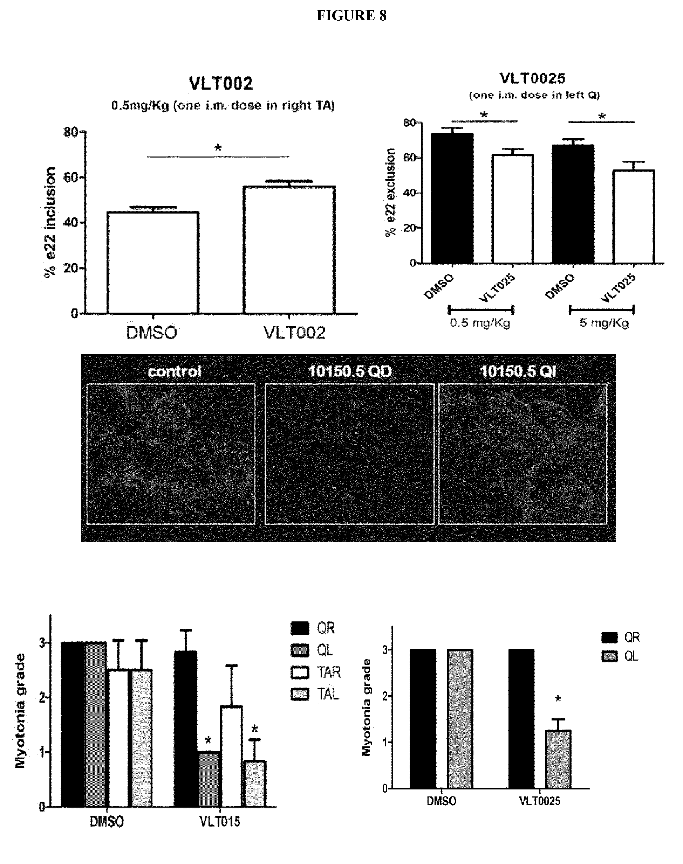

**Figure 8****.** Recovering of disease phenotypes (molecular, histological and functional) in *in vivo* DM1 mice (HSA^{LR}). Upper section: VLT002 and VLT025 suppressed splicing defects in DM1 mouse skeletal muscles. VLT002 (10 µg) and VLT025 (100 µg) were intramuscular injected in the right tibialis anterior (TA) and left quadriceps (Q), respectively, of HSA^{LR} mice that expressed 300 CTG repeats. Semi-quantitative RT-PCR performed three days a/i showed that VLT002 and VLT025 compounds partially reversed splicing defects (exon 22) in muscle transcripts of Serca1 gene (upper section, left and right graphic, respectively). All graphs show the mean and SEM from VLT002 and VLT025-responsive animals. Middle section: Immunofluorescent detection of trans-membrane Clcn1 protein (cell outlines in gray) in quadriceps sections of no-DMl mouse (control) and HSA^{LR} mice without treatment (10150.5 QD-DMSO treated) and after VLT025 treatment (10150.5 QI). In DMSO-treated QD (middle) the Clcn1 signal was almost absent, whereas the fluorescent signal was significantly stronger in the contra-lateral VLT025-treated limb (right, QI-100 µg), similar to non-disease mouse Clcn1 levels (left). Bottom section: Myotonia assessment after high VLT015 and VLT025 dose-treatments (three injections at 60 mg/kg and 75 mg/kg, respectively) showed strong reduction of this functional disease sign from 3 to 1 grade. Contra-lateral DMSO muscles did not display changes in myotonia levels. * indicates p-value < 0.05.

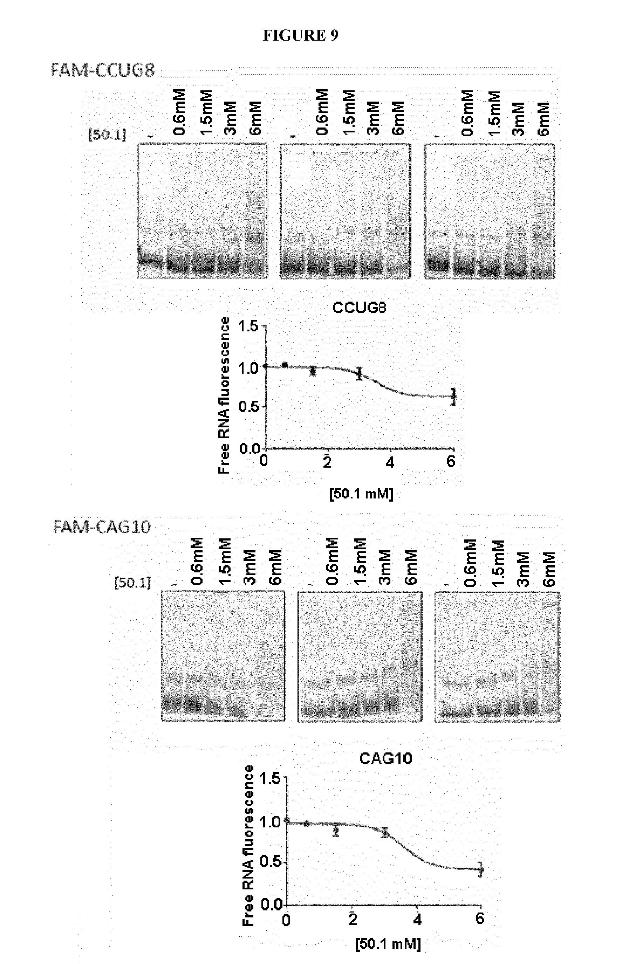

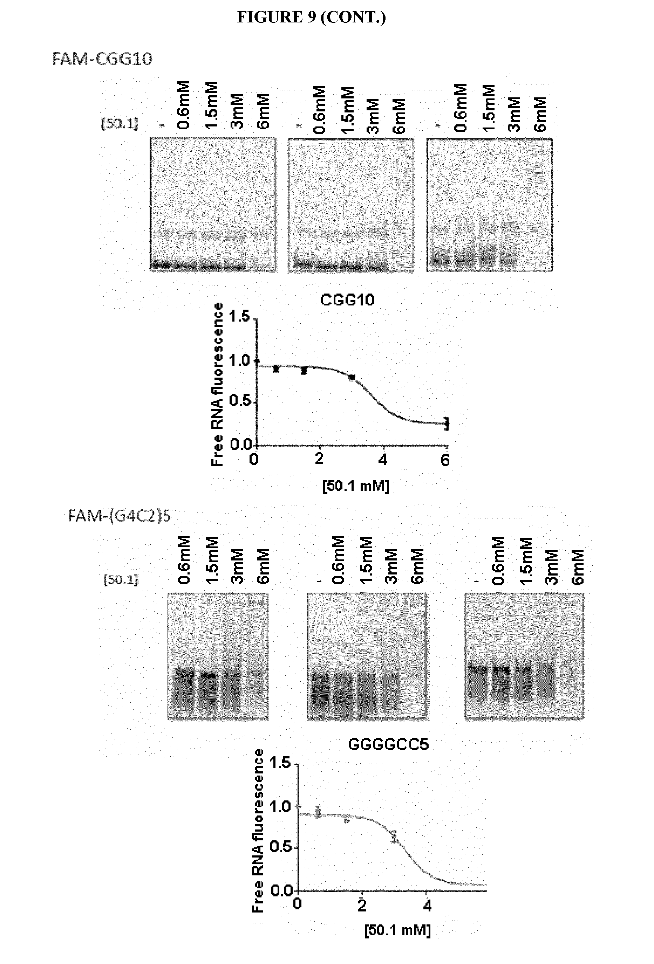

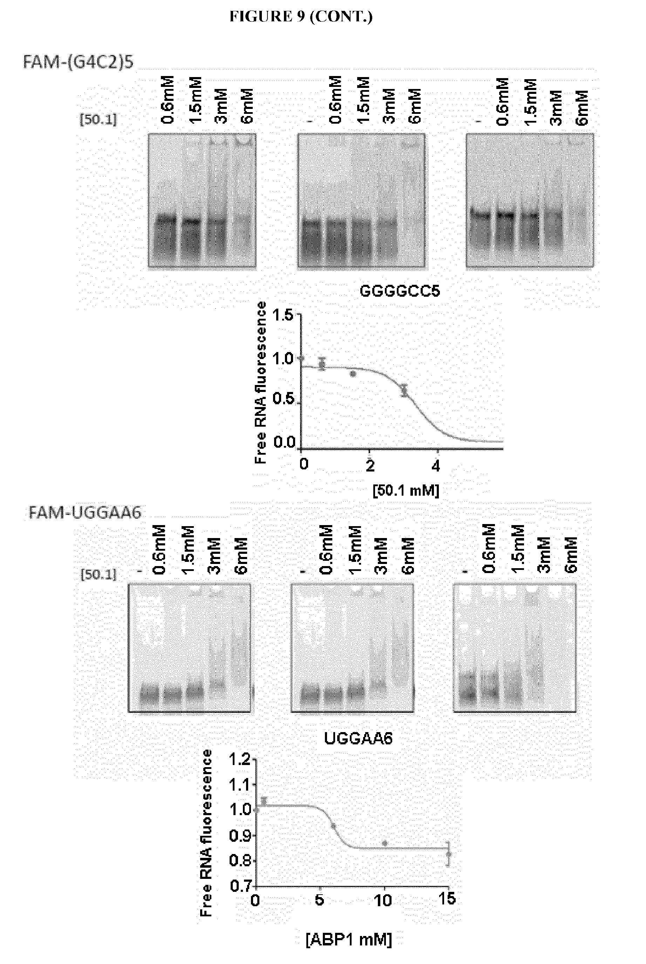

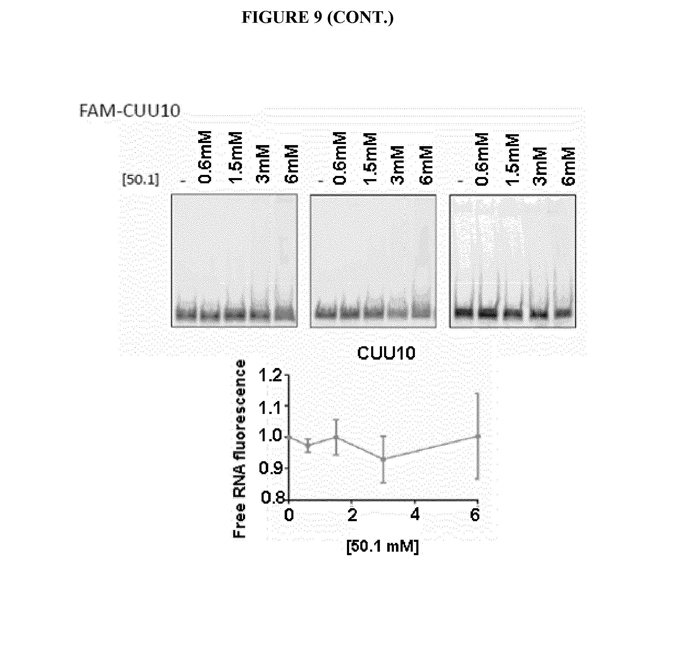

**Figure 9****.** VLT002 binding assessment in additional toxic repeats linked to disease through hairpin formation. VLT002 caused retention of the complex in the well in gel retardation assays after testing binding affinity to CCUG (DM2), CAG (SCA3), CGG (FXTAS), GGGGCC (FTD/ALS) and UGGAA (SCA31) labeled FAM-RNA probes. VLT002 did not bind to FAM-CUU probe, used as negative control, since is described not to form a double-stranded (hairpin) structure, suggesting its binding specificity to RNA-hairpin structures.
This **Figure 9** shows that the compounds of Formula I can be used for treating other diseases having an etiology similar to DM1, because said compounds are not only able to bind CUG repeats (DM1, SCA8, HDL2), but also other nucleotide repeats which are the origin of other diseases such as: CCUG (DM2), CAG (SCA3), CGG (FXTAS), GGGGCC (FTD/ALS) and UGGAA (SCA31).

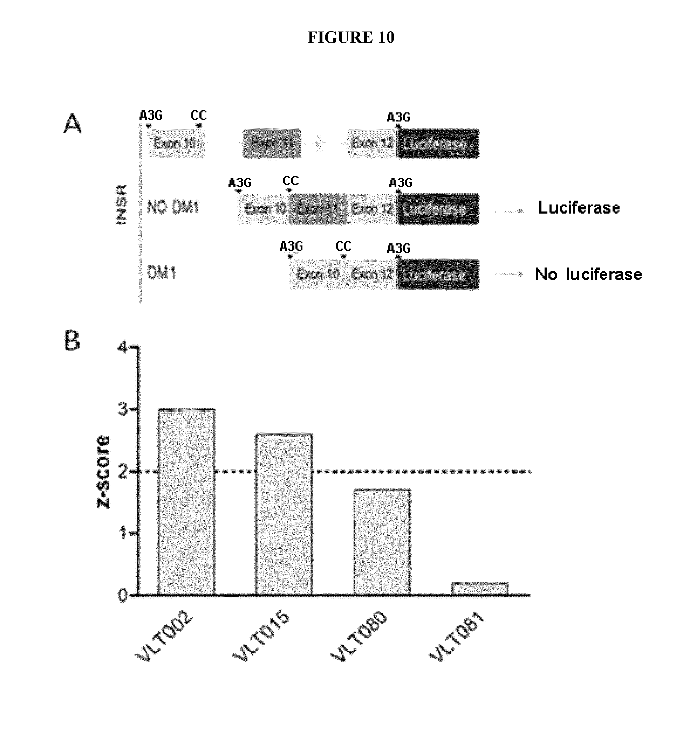

**Figure 10**. **(A)** An in vivo high throughput screening approach was performed by using transgenic flies (*Drosophila*) containing the pathologic CTG expanded repeat (Garcia-Lopez A et al. PLoS One. 3(2):e1595 (2008*)*) and the insulin resistance (IR) minigene (IR-luc assay). IR minigen spans human 10-11-12 exons, aberrantly processed in DM1 patients. As shown by the schematic, the aberrant exclusion of exon 11 is mirroring DM1 conditions (no luciferase reading), being the observed condition after pathologic CUG expression (480 repeats) in transgenic flies. The correct reading frame of the minigene (luciferase reading) is obtained upon inclusion of exon 11, potentially stimulated by the activity of an anti-DM1 small molecule. **(B)** The activity and robustness of the IR-luc assay was tested by performing a validation HTS of ∼15,000 small molecule compounds 96-well format. VLT002 and VLT015 hits chosen, displayed an activity above 2 (z-score value), used as relative activity for positive test compound, compared with compounds with no significant activity (z-score below 2) as VLT080 and VLT081.

### Detailed description of the invention

The present invention is defined in more detail by means of the following examples, whose purpose is illustrating the invention without limiting its scope of protection.

### Example 1. Different approaches used in the present invention to measure anti-DM1 biological activity of the compounds comprised in the Formula I.

**Example 1.1.** After screening identification of the compounds of the invention (see **Figure 10****),** main validation was done using Fluorescence In Situ Hybridization (FISH) detection of aberrant aggregation of ribonuclear foci in cell lines from DM1 patients (see **Figure 1****)** and in DM1 *Drosophila* model (see **Figure 2****)** (Garcia-Lopez et al., Proc. Natl. Acad. Sci. USA. 108(29):11866-71 (2011*)).* Briefly, regarding human cells, it was used MyoD transformed fibroblasts derived from clinically diagnosed DM1 patients (>300 CTG repeats) routinely growing in DMEM supplemented with 10% heat-inactivated fetal bovine serum and maintained at 37°C in a humidified incubator containing 5% CO2. To detect ribonuclear foci, cells were cultured over cover-slides in flat bottomed 24-well plates, after 24h compounds of the invention were added to the cells at desiderate concentration, 24 h later *in situ* hybridization was done by fixing with 4% paraformaldehyde (PFA) 15 min at room temperature, and washed and stored in 70% ethanol. Cells were rehydrated with PBS and pre-hybridized 10 min at room temperature with a pre-hybridization buffer (formamide 30%, 2x saline-sodium citrate buffer (SSC)). Hybridization was performed in a light-proof wet chamber using the same pre-hybridization buffer supplemented with 0.02% bovine serum albumin (BSA), 1 mg/ml yeast tRNA (Sigma), 2 mM sodium (meta)vanadate, 1 mg/ml sperm DNA, 10 % dextran sulfate and 1 ng/µl RNase free HPCL-purified Cy3-labeled (CAG)₁₀ RNA. After hybridization cells were washed 2 x 15 minutes at 45°C with the pre-hybridization buffer and washed with 1X PBS at room temperature. Slides were mounted with Vectashield solution with DAPI and number of foci by cell and number of cells with/without foci (see **Figure 1****)** were counted in a minimum of 100 cells for each treatment and concentration tested with a 63x objective in a Leica microscope. Same FISH approach was performed in *Drosophila* DM1 flies, where thoraces were embedded in OCT and transversal sections were taken with a Leica CM 1510S cryomicrotome. Sections were processed *for in situ* hybridization and counting for number of foci by cell with the same Cy3-labeled (CAG)₁₀ probe (see **Figure 2****).**

All the assayed compounds of Formula I give rise to a statistically significant reduction in the number of foci/nuclei **(****Figures 1** and **2****),** and/or also to a statistically significant increase in the number of cells without foci **(****Figure 1****).**

**Example 1.2.** At the same time human cells were recollected for foci counting. Some (∼150,000-300,000) were prepared for Muscleblind (MBNL1) detection (see **Figure 4****).** After PBS washes, cells were probed overnight, at 4 °C with anti-MBNL1 antibody (1:5000 dilutions, A2764 antibody) in 1xPBS. After washing cells with 1xPBS, cells were incubated with goat anti-rabbit Alexa 488 (1:500 dilution) for 2 h at RT. Cells were washed 2 times, for 10 min at room temperature with 1xPBS and then mounted onto glass slides using a hard-set mounting media that contains DAPI (Vectashield). Cells were imaged on a Leica microscope.

As shown in **Figure 4****,** the compounds of the invention are able to effectively reduce the formation of foci and also to relieve MBNL1 sequestration.

**Example 1.3.** Additional assays involved:
**(A)** Reverse Transcription-PCR (RT-PCR) to detect levels of normal/aberrant splicing events in specific genes. This approach was performed in human cells from patients (see **Figure 5****)** or from DM1 mouse (see **Figure 8** up) (HSA^{LR}, Mankodi A et al., Science, 289:1769-1773 (2000*))* tissues (skeletal muscle), from which total RNA was extracted from ∼0.5-1.5x10⁵ cells using the Zygem Tissue Plus kit. Serca 1, Cypher or cTNNT genes reverse transcription were performed and quantification of normal/aberrant spliced band forms was obtained (ImageJ software) after running PCR products in 2% agarose gels. As shown in **Figures 5** and **8** compounds of the invention are able to recover disease phenotypes.
**(B)** Drosophila Muscle Histology, where five-day old *Drosophila* thoraces were embedded in Epon for transversal, semi-thin sectioning. Muscle area was quantified (see **Figure 3****)** by binarizing images (equal dimensions) and quantifying pixels (%) within muscle tissue with ImageJ software. As can be seen in **Figure 3****,** flies treated with the compounds of the invention displayed a significant increase of the muscle area relative to DMSO-treated flies (quantified in graphic) * indicates p-value < 0.05. A minimum of 8 flies were analyzed in each case.

**Example 1.4.** Also, an immunohistochemistry approach was used to measure ClC-1 protein levels (see **Figure 8** middle) in mouse tissues (skeletal muscle). Briefly, frozen sections of tibialis anterior & quadriceps muscle were prepared for routine immunostaining by using antibody against the ClC-1 protein (Alpha Diagnostic, San Antonio, TX). Primary antibody concentration used was 1:50 for ClC-1. Secondary antibody was goat anti-rabbit Alexa Fluor 488 and 546 (Invitrogen) at 1:400. Stacks of Z plane images were processed by using a blind point spread function. For comparison of injected and non-injected muscle, sections were stained on the same slide, and images were obtained and processed under the same exposure and threshold settings.

**Example 1.5.** Myotonia, as important functional DM1 sign displayed by HSA^{LR} mice was measured by electromyography **(****Figure 8** bottom). EMG analysis of myotonia was performed under general anesthesia by blinded examiners following 10 electrode insertions in each muscle on study and counting the audible myotonic discharges. Myotonia was graded as follows: 0 indicates no myotonia; 1, occasional myotonic discharge in less than 50% of electrode insertions; 2, myotonic discharge in greater than 50% of insertions; 3, myotonic discharge with nearly every insertion. WT mice do not show myotonia by these methods.

### Example 2. Assays used for the establishment of mechanism of action suggested for the compounds of the invention.

**Example 2.1.** Fluorescence Electrophoretic Mobility Shift Assay to test RNA-compound binding. Briefly, an aliquot of carboxyfluorescein (FAM)-CUG10 RNA (DM1, HDL2, SCA8; see **Figure 6****)** was heated at 70 °C for 5 min and diluted to 300 nM. The binding reaction with compounds was then carried out as described in (Garcia-Lopez A et al. Proc. Natl. Acad. Sci. USA. 108(29):11866-71 (2011*)).* The reaction mix was loaded onto 8% polyacrylamide nondenaturing gels, and electrophoresis was conducted at 4 °C in TB 1X for approximately 30 min at 240V, followed by fluorescence visualization. Quantification of the free RNA band intensity was carried out using the ImageJ software. A non-linear regression analysis was used to calculate EC50, based on a total of three experimental replicates. Same approach was developed with (FAM)-CCUG8 (DM2), CAG10 (SCA3), CGG10 (FXTAS), GGGGCC5 (ALS/FSD), UGGAA6 (SCA31) probes (see **Figure 9****).**

**Example 2.2.** Aminopurine Fluorescence assay to detect hairpin double strand RNA unfolding. An aliquot of 2-aminopurine (2AP)-CUG23 RNA (1 µM in binding buffer) was heated at 90 °C for 3 min, placed on ice, and further diluted to 30 nM in a final volume of 1 mL in binding buffer. The fluorescence emission spectra of the RNA were obtained as described in ((Garcia-Lopez A et al. Proc. Natl. Acad. Sci. USA. 108(29):11866-71 (2011*)),* monitoring fluorescence emission peaks at 363 nm and 375 nm. All measures were taken in a Shimadzu RF-1501 spectrofluorophotometer. As shown in **Figure 7****,** compounds of the invention are able to modify the hairpin loops formed by the CUG repeats.

### Example 3. Obtaining of the compound of Formula I from Total Synthesis. Process for the preparation of the "aporphines", precursor of general Formula I.

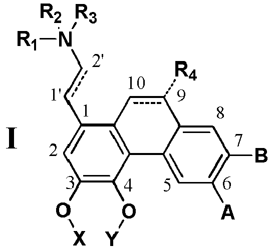

**R1, R2, R3** = H or CH₃ or CH₂CH₃ or CH₂CH₂CH₃ or Benzyl
**R₄** = H or OH
**X, Y**= H or CH₂ or CH₃ or C₇H₇ or C₂H₄Br or C₃H₆NO or C₇H₅NF or C₇H₃NSF₃ or CH₂CH₃
**A, B** = H or OCH₂O or Cl or OCH₃ or OC₃H₇ or OC₇H₇ or OC₂H₄Br or OC₃H₆NO or OC₇H₅NF or OC₇H₃NSF₃ or OCH₂CH₃ or OH
a) Starting material: isovanillin: 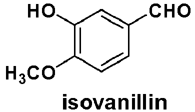
b) Reagents: benzyl chloride, K₂CO₃/EtOH, reflux; and nitromethane, NH₄OAc/AcOH, reflux: 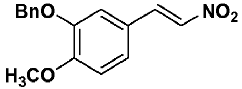
c) Reagents: AlLiH₄, ether/THF, reflux: 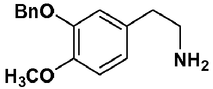
d) Reagents: phenylacetyl chloride derivatives, CH₂Cl₂/NaOH 5 %: 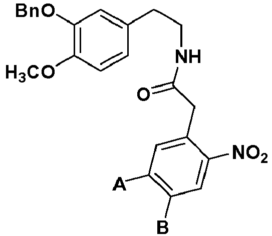
e) Reagents: EtOH/HCl, reflux; BBr₃, CH₂Cl₂, -78 °C; CH₂Cl₂/CsF/DMF reflux: 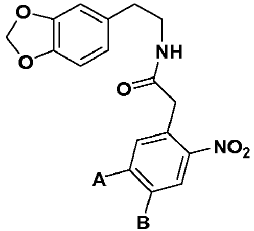
f) Reagents: POCl₃, CH₂Cl₂ reflux; NaBH₄, MeOH, rt; benzyl chloride, K₂CO₃/EtOH, reflux: 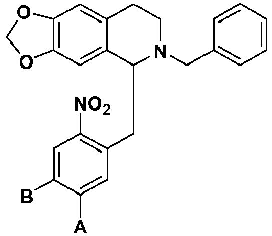
g) Reagents: Zn/HCl reflux: 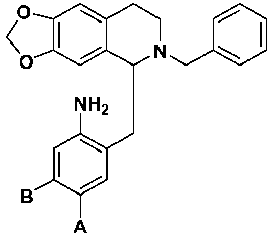
h) Reagents: NaNO₂, H₂SO₄/AcOH; Cu, Cu₂Cl₂, reflux: 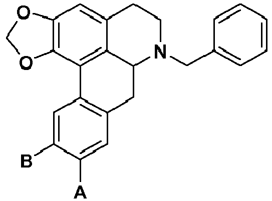
i) Reagents: EtOH/HCl, reflux:

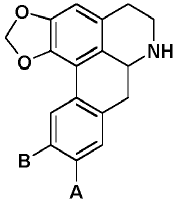

### Example 4. Obtaining of the compounds of Formula I from natural source. General procedure for isolation of VTL040 and VTL015.

Plant Material: The concentrated methanolic extract of the bark of *Xylopia* spp (Annonaceae), or leaves of *Berberis* spp (Berberidaceae) (3 g), was dissolved in methanol / HCl 5 % 1:9. The mixture was heated and stirred, and then filtered. The aqueous-methanolic extract was partially evaporated and basified with NH₄OH until pH=9. Then the solution was washed with CH₂Cl₂ (15 ml) up to five times. The organic layers were evaporated to dryness (385 mg, 13 %) and a column chromatography over silica gel flash was eluted with toluene-EtOAc-MeOH-TEA 6:2.5:1.5:0.1. 60 mg (2 %) of **VLT040** were obtained. The procedure was repeated as many times as necessary.

**General Instrumentation.** Melting points were taken on a Cambridge microscope instruments coupled with a Reichert-Jung. EI and FAB mass spectra were recorded on a VG Auto Spec Fisons spectrometer instruments (Fisons, Manchester, United Kingdom). ¹H NMR and ¹³C NMR spectra were recorded with CDCl₃ as solvent on a Bruker AC-300, AC-400 or AC-500. Multiplicities of ¹³C NMR resonances were assigned by DEPT experiments. NOE DIFF irradiations, COSY, HMQC, HSQC and HMBC correlations were recorded at 400 MHz and 500 MHz (Bruker AC-400 or AC-500). All reactions were monitored by analytical TLC with silicagel 60 F₂₅₄ (Merck 5554). The residues were purified through silica gel 60 (40-63 µm, Merck 9385) column chromatography. Solvents and reagents were used as purchased from commercial sources. Quoted yields are of purified material. The HCl salts of the synthesised compounds were prepared from the corresponding base with 5% HCl in MeOH.

**VLT040**. ¹H NMR* (300 MHz, CDCl₃): δ= 8.00 (d, 1H, *J*= 8, H-11), 7.28-7.11(m, 3H, H-8, H-9, H-10), 6.48 (s, 1H, H-3), 5.96 and 5.82 (2d, 2H, *J*= 1.2 Hz, OCH₂O), 3.90 (m, 1H, H-6a), 3.30 (m, 1H, H-5α), 3.00 (m, 1H, H-4α), 2.95 (m, 1H, H-5β), 2.90 (m, 1H, H-7α), 2.80 (m, 2H, H-4β and H-7β); ¹³C NMR* (75 MHz, CDCl₃): δ= 146.8 (C-2), 142.5 (C-1), 135.4 (C-7a), 131.0 (C-11a), 128.7 (C-4a), 128.1 (CH-1b), 127.5 (CH-8), 127.2 (CH-10), 127.1 (CH-9), 127.0 (CH-11), 116.3 (C-1a), 108.0 (CH-3), 100.6 (OCH₂O), 53.6 (CH-6a), 43.6 (CH₂-5), 37.4 (CH₂-7), 29.6 (CH₂-4); *The assignments were made by COSY 45, DEPT, HSQC and HMBC.

**VTL015.** ¹H NMR* (300 MHz, CDCl₃): δ= 7.89 (s, 1H, H-11), 6.90 (s, 1H, H-8), 6.60 (s, 1H, H-3), 3.92 (s, 3H, OCH₃-10), 3.61 (s, 3H, OCH₃-1), 3.2-2.9 (m, 4H, H-6a, H-4α, H-5α, H-7-α), 2.70-2.40 (m, 3H, H-4β, H-5β, H-5β), 2.58 (s, 3H, *N*-CH₃); ¹³C NMR* (75 MHz, CDCl₃): δ= 148.1 (C-10), 145.6 (C-1), 145.0 (C-2), 142.2 (C-9), 130.0 (C-7a), 129.3 (CH-4a), 126.2 (C-1a), 126.1 (C-1b),123,5 (C-11a), 114.4 (CH-8), 113.4 (CH-3), 110.5 (CH-11), 62.4 (CH-6a), 60.2 (OCH₃-1), 56.2 (OCH₃-10), 53.2 (CH₂-5), 43.8 (*N*-CH₃), 33.8 (CH₂-7), 28.4 (CH₂-4); *The assignments were made by COSY 45, DEPT, HSQC and HMBC.

### Example 5. Semisynthesis of Derivatives.

### A. General process for the preparation of the general Formula I as derivatives from aporphines:

a) Starting material: "VLT040", aporphine alkaloid synthetized or isolated from *Xylopia ligustifolia* (Annonaceae); and VTL015, aporphine alkaloid synthetized or isolated from *Peumus boldus* (Monimiaceae). 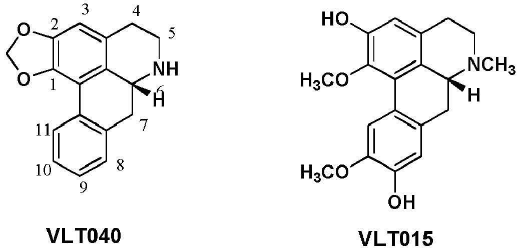
b) Reagents: Methyl iodide, MeOH/ether. 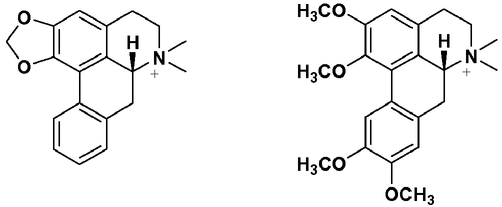
c) Reagents: KOH 3N, MeOH: 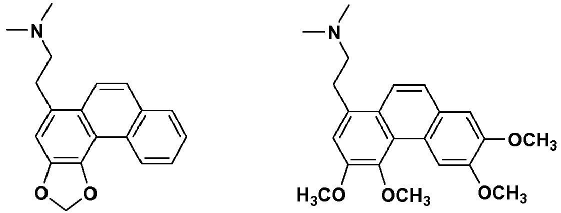
d) Reagents: Methyl iodide, MeOH/ether:

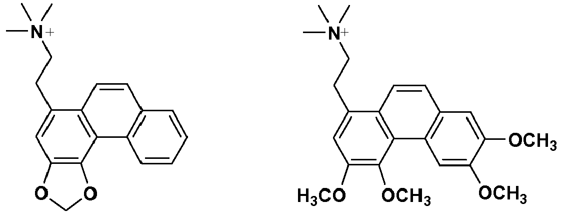

### B. General procedure for N,N-dimethylation of aporphine.

The appropriate aporphine, e.g., **VLT040** (pm= 265, 80 mg, 0.3 mmol) was dissolved in 0.3 ml of MeOH and 5.5 ml of Et₂O; 1.3 ml of iodomethane was added, and the mixture stirred at room temperature for 6 hours. The reaction mixture was evaporated to dryness and redissolved in 2 ml of MeOH; 30 ml of Et₂O were added and the suspension was centrifuged for five minutes at 1500 rpm speed. The supernatant was removed and the pellet redissolved in 2 ml of MeOH. This procedure was repeated as necessary. The pellet was taken and evaporated to dryness to afford 85 mg of *N*,*N*-dimethyl-VLT040 (pm= 294, 0.28 mmol, 93 %) ***N,N*-dimethyl-VLT040 (II.1).**

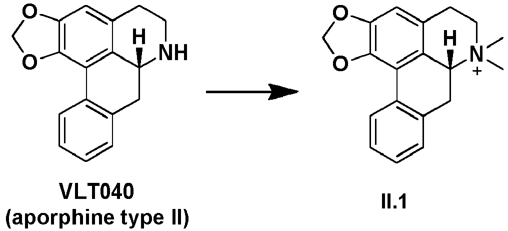

### C. General procedure for aporphine Hofmann's elimination: I.1 (VLT002)

Formation of the phenanthrenic alkaloids was carried out under Hofmann's conditions using the quaternary ammonium salt of the corresponding aporphine, e.g., ***N,N-*dimethyl-VLT040,II.1** (85 mg, pm= 294, 0.28 mmol), which is dissolved in 20 ml of MeOH,; 20 ml of KOH 3N were added. The mixture was stirred at 45°C for eight hours. After partial evaporation of organic solvent, the aqueous solution is extracted with CH₂Cl₂. The organic layers were dried with Na₂SO₄ and concentrated to dryness. The residue obtained was purified by a silica gel flash column (CH₂Cl₂-MeOH 95:5) to afford 60 mg of phenantrenic alkaloid **I.1** (pm= 293, 0.20 mmol, 73 %). **Stephenanthrine, I.1 (VLT002).** ¹H NMR* (300 MHz, CDCl₃): δ= 9.06 (d, 1H, H-5, *J*=8), 7.86 (d, 1H, H-10, *J*=9), 7.82 (m, 1H, H-8), 7.62-7.42 (m, 3H, H-6, H-7, H-9), 7.17 (s, 1H, H-2), 6.15 (s, 2H, OCH₂O), 3.35 (t, 2H, CH₂-β), 2.85 (t, 2H, CH₂-α), 2.54 (s, 6H, *N*-(CH₃)₂);¹³C NMR* (75 MHz, CDCl₃): δ= 145.0 (C-3), 142.2 (C-4), 132.0 (C-8a), 130.8 (C-1), 128.8 (C-4b), 127.6 (CH-8), 127.3 (CH-5), 126.7 (CH-7), 126.2 (CH-6), 126.0 (C-10a), 125.1 (CH-9), 122.7 (C-10), 117.1 (C-4a), 110.5 (CH-2), 101.1 (OCH₂O), 61.2 (CH₂-α), 45.3 (*N*(CH₃)₂), 31.9 (CH₂-β); *The assignments were made by COSY 45, DEPT, HSQC and HMBC; ESMS m/z (%): 294 (100) [M+1]⁺.

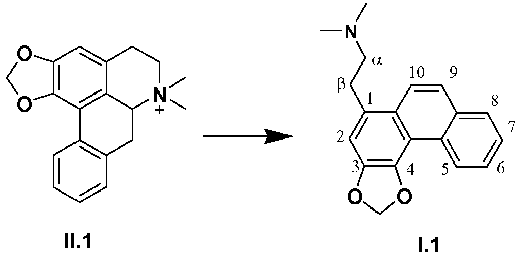

### D. General procedure for aporphine methylation and ring opening: I.2 (VLT024)

A mixture of **VTL015** (pm= 327, 150 mg, 0.45 mmol), iodomethane (6 eq, 1.83 mmol, 0.12 ml) and anhydrous K₂CO₃ (4 eq, 1.83 mmol, 126 mg) in dry acetone (15 ml) was stirred and refluxed for 24 hours. The solution was concentrated to dryness and extracted with CH₂Cl₂ (20 ml x 3). The organic layers were washed with brine and concentrated to dryness to afford 122 mg of the resulting product **I.2 (VLT024)** (pm= 384, 0.31 mmol, 70 %). **I.2** ( **VLT024).** ¹H NMR* (300 MHz, DMSO-d₆): δ= 9.15 (s, 1H, H-5), 7.82 (d, 1H, H-10, *J*= 10), 7.78 (d, 1H, H-9, *J*= 10), 7.50 (s, 1H, H-8), 7.43 (s, 1H, H-2), 4.05, 3.99, 3.93 and 3.86 (4s, 12H, 4-OCH₃), 3.75-3.57 (m, 4H, CH₂-α, CH₂-β), 3.30 (s, 9H, 3-NCH₃); ¹³C NMR* (75 MHz, CDCl₃): ESMS m/z (%): 384 (100) [M]⁺.

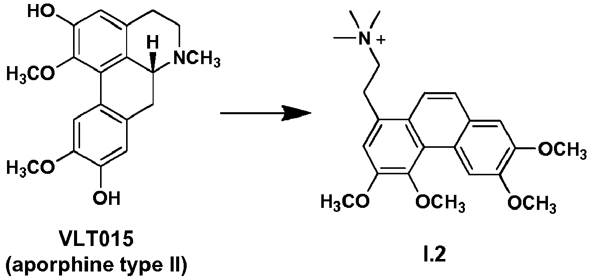

### E. General procedure for aporphine benzylation and ring opening: I.3 (VLT026)

A mixture of the aporphine, e.g, **VTL015** (pm= 327, 250 mg, 0.76 mmol), benzyl chloride (3 eq, 2.28 mmol, 0.26 ml) and anhydrous K₂CO₃ (1.5 eq, 1.14 mmol, 157 mg) in EtOH (10 ml) was refluxed overnight. After being stirred, the reaction mixture was concentrated to dryness and redissolved in 10 ml of CH₂Cl₂, and then 5% aqueus NaOH (3 x 10 ml) was added. The organic layer was washed with brine (2 x 10 ml) and H₂O (2 x 10 ml), dried with anhydrous Na₂SO₄ and evaporated to dryness. The residue obtained was purified by a silica gel flash column (toluene-EtOAc-MeOH-TEA 8:1:1:0.1) to afford the resulting compound (**I.3**) (90 mg; pm= 597, 0.15 nmol; 20 %). **I.3 (VLT026).** ¹H NMR* (300 MHz, CDCl₃): δ= 9.3 (s, 1H, H-11),7.8 (d, 1H, H-10), 6.6 (s, 1H, H-3), 7.6-7.2(m, 16 H -OBn, -NBn, H-9), 7.2 (s, 1H, H-8), 5.4 (2H, OCH₂Ph), 5.3 (OCH₂Ph), 4.1 (s, 3H, -OCH₃), 4.0 (s, 3H, -OCH₃), 3.6 (s, 2H, NCH₂Ph), 3.2 (t, 2H, CH₂-α), 2.8 (t, CH₂-β), 2.4 ppm (s, 3H, NCH₃); ¹³C NMR* (75 MHz, CDCl₃): δ=; ESMS *m*/*z* (%): 598 (100)[M+1]⁺.

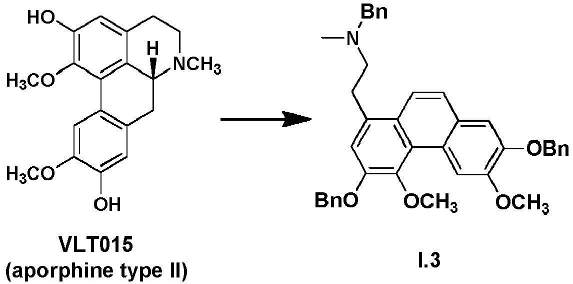

## Claims

1. Compounds of Formula I, derivatives, or pharmaceutically acceptable salts thereof: 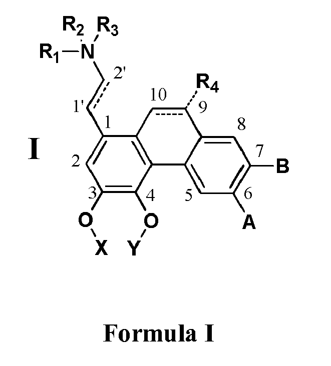 wherein
**R₁**, **R₂** and **R₃** may be, independently: H, CH₃, CH₂CH₃, CH₂CH₂CH₃ or Benzyl;
**R₃** may be C10, as a part of a cycling ring with subsequent lack of double bond C9-C10;
**R₄** may be, independently: H, O or OH;
**X** and **Y** may be, independently: H, CH₂, CH₃, CH₂CH₃, Benzyl, C₂H₄Br, C₃H₆NO, C₇H_{S}NF or C₇H₃NSF3; or a part of a cyclic ring, comprising X=Y;
**A** and **B** may be, independently: H, OH, OCH₂O, Cl, OCH₃, OC₃H₇, OC₇H₇, OC₂H₄Br, OC₃H₆NO, OC₇H₅NF or OC₇H₃NSF₃.
for use in the prevention and/or treatment of human diseases involving the presence of toxic RNA comprising: DM1, DM2, SCAs, FXTAS, HDL2 or FTD/ALS.

2. Compounds of Formula I, according to claim 1, for use as an antimyotonic agent.

3. Compounds of Formula I, according to claim 1, selected from: 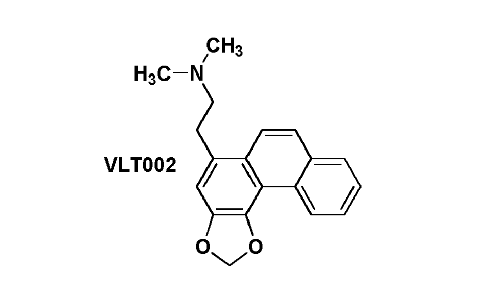 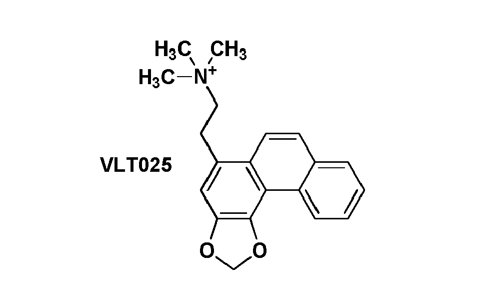 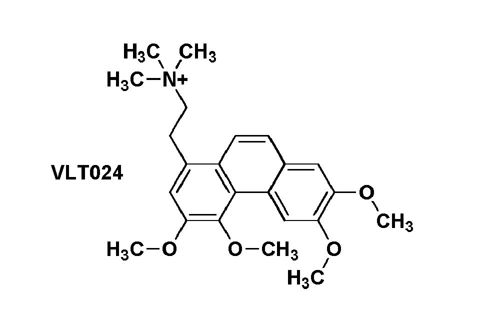 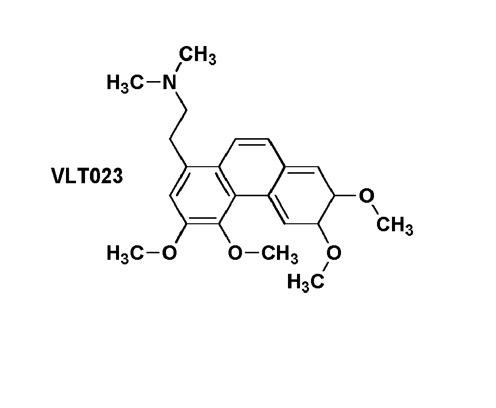 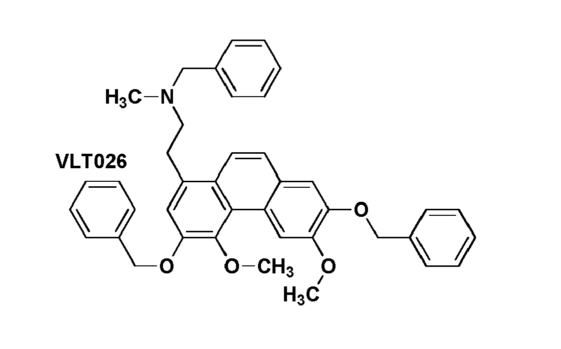 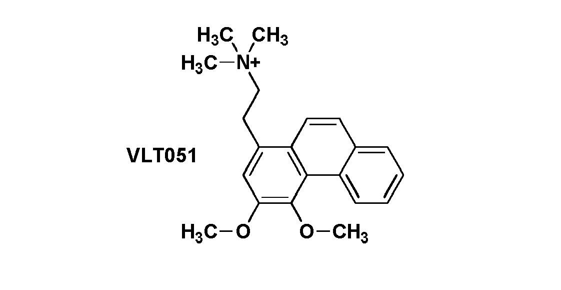 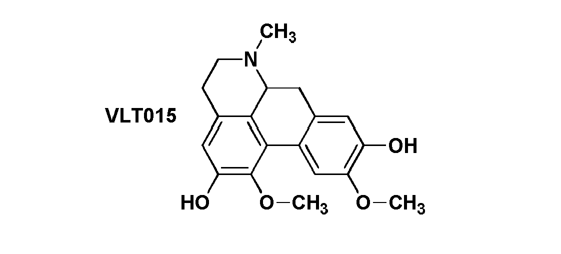 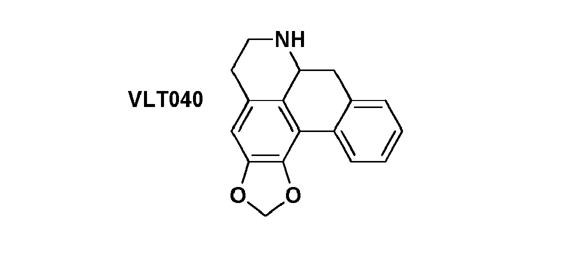 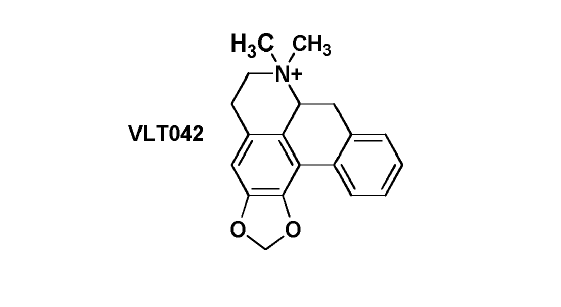 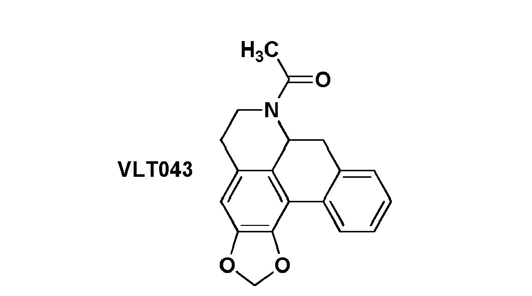 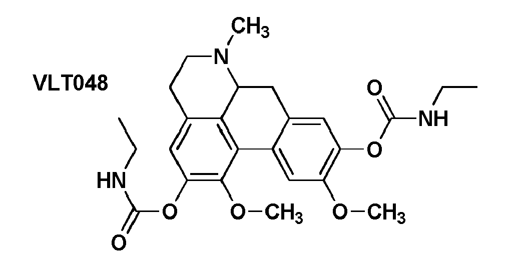 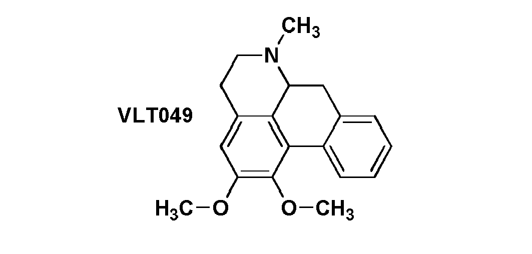 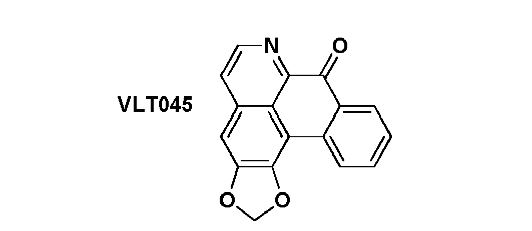 for use in the prevention and/or treatment of human diseases involving the presence of toxic RNA comprising: DM1, DM2, SCAs, FXTAS, HDL2 or FTD/ALS.

4. Compounds of Formula I, according to claim 1, selected from: 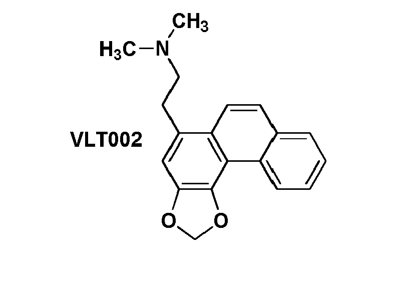 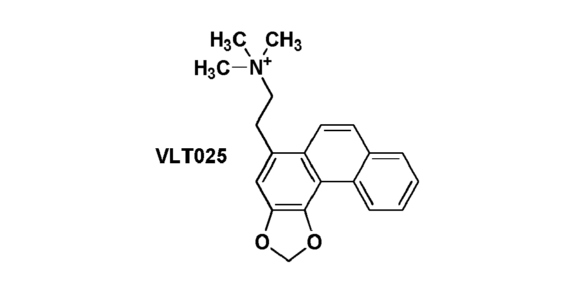 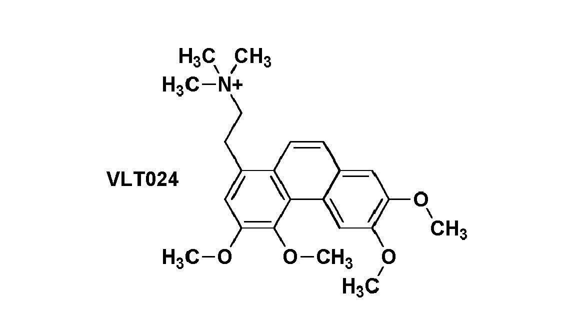 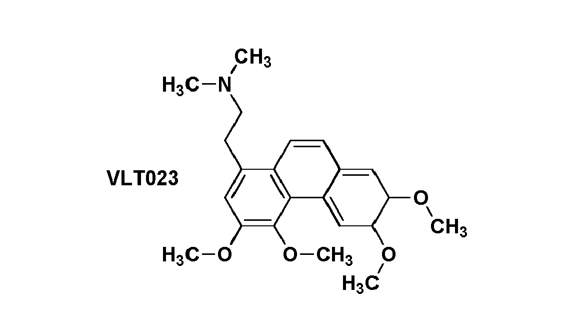 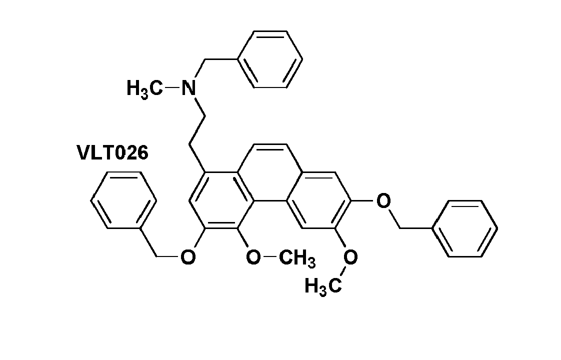 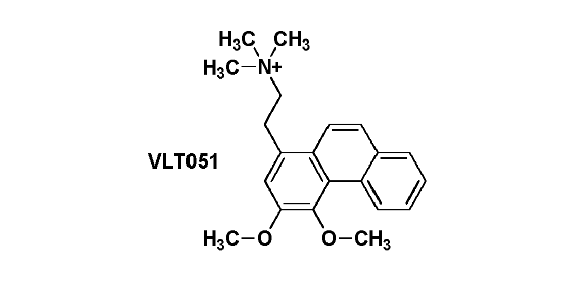 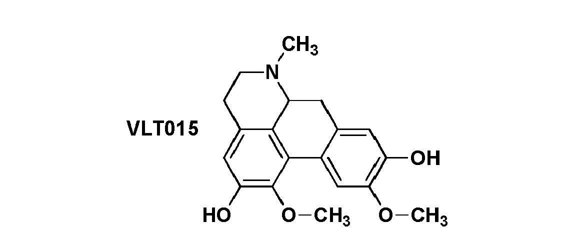 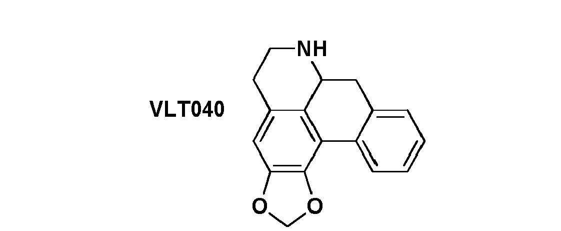 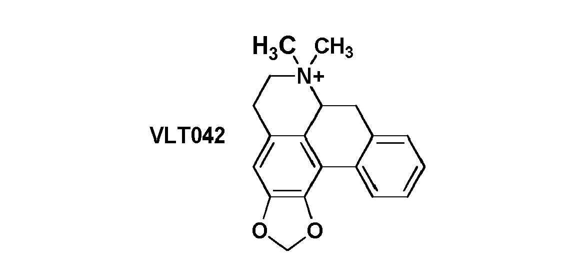 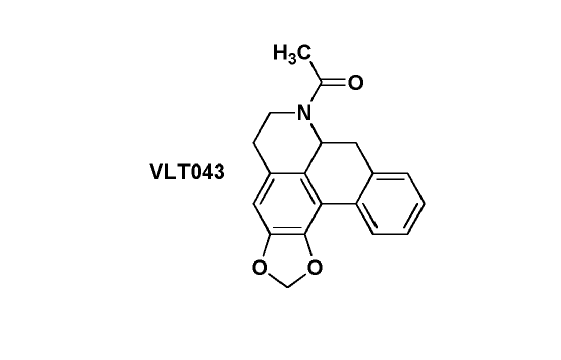 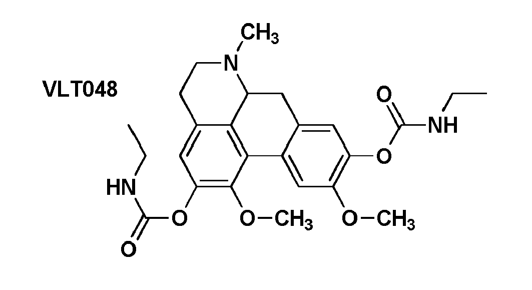 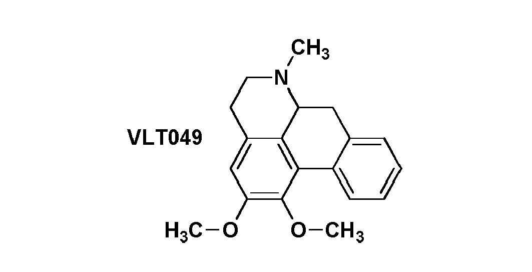 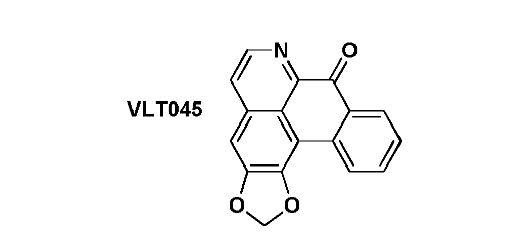 for use as a an antimyotonic agent.

5. Compounds of Formula I, according to claim 1, selected from: 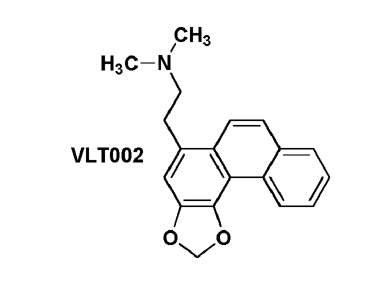 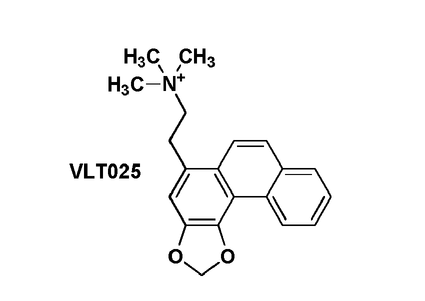 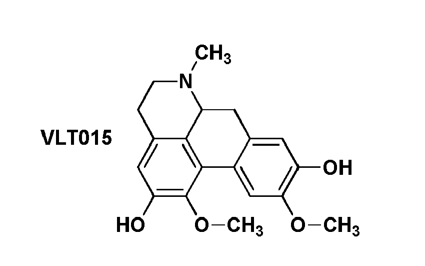 for use in the prevention and/or treatment of human diseases involving the presence of toxic RNA comprising: DM1, DM2, SCAs, FXTAS, HDL2 or FTD/ALS.

6. Compounds of Formula I, according to claim 1, selected from: 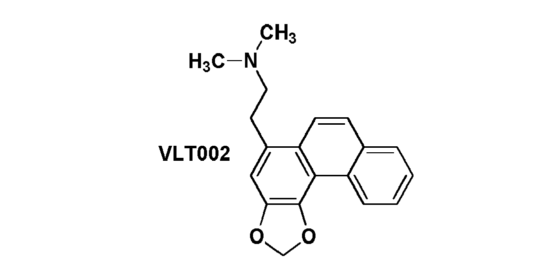 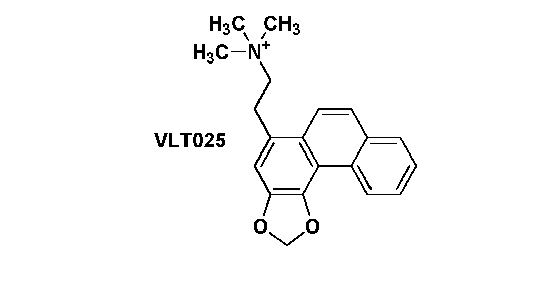 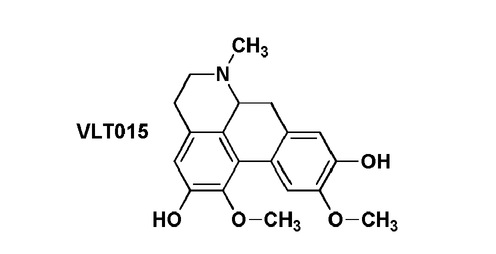 for use as an antymiotonic agent.

7. Pharmaceutical composition comprising a compound of Formula I and pharmaceutically acceptable carriers, for use in the prevention and/or treatment of human diseases involving the presence of toxic RNA comprising: DM1, DM2, SCAs, FXTAS, HDL2 or FTD/ALS.

8. Pharmaceutical composition comprising a compound of Formula I and pharmaceutically acceptable carriers, according to claim 7, for use as an antimyotonic agent.

9. Compound of Formula I selected from:
